# EUROPEAN PATENT SPECIFICATION

(11) **EP 1 635 835 B1**
(45) Date of publication and mention of the grant of the patent: **06.01.2010**
(21) Application number: 04739809.4
(22) Date of filing: 11.06.2004
(51) Int. Cl.: A61K 31/505, C07D 239/42, C07D 401/04, C07D 401/14, C07D 403/04, C07D 417/04, A61K 31/506, A61P 35/00

(54) **2-AMINOPYRIMIDINE DERIVATIVES AS RAF KINASE INHIBITORS**
2-AMINOPYRIMIDIN-DERIVATE ALS RAF-KINASE-HEMMER
DERIVES DE 2-AMINOPYRIMIDINE UTILISES COMME INHIBITEURS DE RAF KINASE

(30) Priority: 13.06.2003 US 478709 P
(43) Date of publication of application: 22.03.2006
(73) Proprietor: Novartis AG, 4056 Basel (CH); Novartis Pharma GmbH, 1230 Wien (AT)
(72) Inventor: BATT, David, Bryant, Wayland, MA 01778 (US); RAMSEY, Timothy, Michael, Weston, MA 02493 (US); SABIO, Michael, Lloyd, Randolph, NJ 07869 (US)
(74) Representative: Gilholm, Stephen Philip
(86) International application number: PCT/EP2004/006317
(87) International publication number: WO 2004/110452

(56) References cited:
- WO-A-02/22597
- WO-A-02/093164
- WO-A-03/022833
- WO-A-03/062220
- WO-A-03/066613
- WO-A-03/070173
- US-B1- 6 180 631
- ZIMMERMANN J ET AL: "Phenylamino-pyrimidine (PAP) - derivatives: a new class of potent and highly selective PDGF-receptor autophosphorylation inhibitors" BIOORGANIC & MEDICINAL CHEMISTRY LETTERS, OXFORD, GB, vol. 6, no. 11, 4 June 1996 (1996-06-04), pages 1221-1226, XP004134858 ISSN: 0960-894X
- TANIS, KEITH Q. ET AL: "Two distinct phosphorylation pathways have additive effects on Abl family kinase activation" MOLECULAR AND CELLULAR BIOLOGY , 23(11), 3884-3896 CODEN: MCEBD4; ISSN: 0270-7306, June 2003 (2003-06), XP002292218
- RACHID, ZAKARIA ET AL: "Synthesis of pyrimidinopyridine-triazene conjugates targeted to abl tyrosine kinase" BIOORGANIC & MEDICINAL CHEMISTRY LETTERS , 13(19), 3297-3300 CODEN: BMCLE8; ISSN: 0960-894X, 2003, XP002292219

## Description

### Summary

The present invention relates to the discovery that certain compounds inhibit RAF kinase, a serine/threonine kinase that functions in the MAP kinase signaling pathway, and to the use of the compounds for the treatment of diseases characterized by excessive signaling in the MAP kinase signaling pathway, for example proliferative diseases like certain cancers.

### Background

Cells communicate various aspects of their extracellular environment to the nucleus by using various signal transduction pathways. Many of these signals are transmitted by protein kinases which activate various factors through the transfer of phosphate groups. Disruption of signal transduction by inhibiting appropriate kinase activity can have a clinical benefit as has been demonstrated by imatinib, an inhibitor of bcr-abl kinase, which is marketed as its mesylate salt under the brand GLEEVEC (in the United States) or GLIVEC.

The MAP kinase signaling pathway is known in the art as one of the pathways for growth factors to send their signal to proliferate from the extracellular environment to the cell nucleus. The growth factors activate transmembrane receptors located on the cell surface which in turn start a cascade whereby RAS is activated and recruits RAF kinase to the membrane where it is activated and in turn activates MEK kinase which then activates ERK kinase. Activated ERK kinase can move to the nucleus where it activates various gene transcription factors. Aberrations in this pathway can lead to altered gene transcription, cellular growth and contribute to tumorogenicity by negatively regulating apoptosis and transmitting proliferative and angiogenic signals. Inhibitors of RAF kinase have been shown to block signaling through the MAP kinase signaling pathway.

The RAF kinase family is known to have three members designated C-RAF, also known as RAF-1, B-RAF and A-RAF. It has been reported that B-RAF kinase is commonly activated by one of several somatic point mutations in human cancer, including 59% of the melanoma cell lines tested. See, Davies, H. et al, Nature 417, 949-954 (2002). This invention relates to the discovery of a class of compounds that efficiently inhibit one or more members of the RAF kinase family.

The RAF kinase inhibiting property of the compounds makes them useful as therapeutic agents for the treatment for proliferative diseases characterized by an aberrant MAP kinase signaling pathway, particularly many cancers characterized by overexpression of RAF kinase or an activating mutation of RAF kinase, such as melanoma having mutated B-RAF, especially wherein the mutated B-RAF is the V599E mutant. The present invention also provides compounds for treating other conditions characterized by an aberrant MAP kinase signaling pathway, particularly where B-RAF is mutated, for example benign Nevi moles having mutated B-RAF, with the compounds.

### Description

The present invention relates to compounds of formula (I) and to their use for preparing medicaments useful for treating a patient having a disease characterized by excessive signaling through the MAP kinase pathway, which comprises administering to the patient an effective RAF kinase inhibiting amount of a compound of formula (I).

The inventive compounds are described by formula (I) 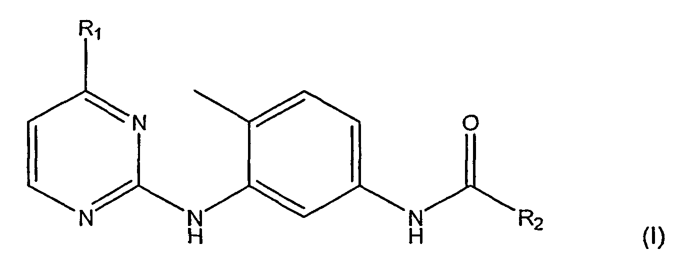 wherein
R₁ is a phenyl radical that is unsubstituted or substituted by one substituent or a heteroaryl radical selected from a thiazolyl radical, a pyrazinyl radical and a pyrimidinyl radical, each of which is unsubstituted or substituted by one substituent, or is a 6-substituted, 3-pyridyl; and R₂ is phenyl that is substituted in the 3-position by fluorine, halo-lower alkyl, halo-lower alkoxy or halo-lower alkylthio;
or an N-oxide or a pharmaceutically acceptable salt thereof.

Within the context of the present disclosure, the general terms used herein to describe compounds of formula (I) have the following meanings, unless indicated otherwise.

The term "lower" when referring to substituents such as alkyl, alkoxy, alkyl amine, alkylthio and the like denotes a radical having up to and including a maximum of 7, especially from 1 up to and including a maximum of 4, carbon atoms, the radicals in question being unbranched or branched one or more times.

The alkyl portion of lower alkyl, lower alkoxy, mono- or di-lower alkyl amino, lower alkyl thio and other substituents with an alkyl portion is especially C₁-C₄alkyl, for example n-butyl, sec-butyl, tert-butyl, n-propyl, isopropyl, methyl or ethyl. Such alkyl substituents are unsubstituted or substituted by halogen, hydroxy, nitro, cyano, lower alkoxy, C₃-C₇ cycloalkyl, amino, or mono- or di-lower alkyl amino, unless otherwise indicated.

Halo-lower alkyl, halo-lower alkyloxy, halo-lower alkylthio and the like refer to substituents having an alkyl portion wherein the alkyl portion is mono- to completely substituted by halogen. Halo-lower alkyl, halo-lower alkyloxy, halo-lower alkylthio and the like are included within substituted lower alkyl, substituted lower alkoxy, substituted lower alkylthio and the like.

Halogen is especially fluorine, chlorine, bromine or iodine, more especially fluorine, chlorine or bromine, in particular fluorine.

Any reference to compounds, salts and the like in the plural is always to be understood as including one compound, one salt or the like.

A phenyl radical is generally an unsubstituted phenyl or phenyl that is substituted with from 1-5, preferably 1 or 2, substituents. Appropriate substituents include, but are not limited to, amino, mono- or di-lower alkyl substituted amino, wherein the lower alkyl substitents may be unsubstituted or further substituted by those substitutents listed above for alkyl groups, halogen, lower alkyl, substituted lower alkyl, hydroxy, lower alkoxy, substituted lower alkoxy, nitro, cyano, mercapto, lower alkylthio, halo-lower alkylthio, heterocyclyl, heteroaryl, heterocyclylalkyl, heteroarylalkyl, lower alkanoyl, carbamoyl, and N-mono- or N,N-di-lower alkyl substituted carbamoyl, wherein the lower alkyl substitents may be unsubstituted or further substituted.

R₁ as a phenyl radical is especially unsubstituted phenyl or phenyl which is substituted by one substituent. Especially important substituents for R₁ phenyl radicals include amino, mono- or di-lower alkyl amino, where the alkyl groups are unsubstituted or substituted, halogen, lower alkyl, substituted lower alkyl, hydroxy, lower alkoxy, substituted lower alkoxy, nitro, cyano, mercapto, lower alkylthio and substituted lower alkylthio.

R₁ as a phenyl radical is especially phenyl that is unsubstituted or substituted by one substituent selected from halogen, especially fluorine or chlorine; lower alkyl, especially methyl, ethyl, propyl or t-butyl; halo-lower alkyl, especially trifluoromethyl; hydroxy; lower alkoxy, especially methoxy or ethoxy; halo-lower alkyloxy, such as trifluoromethoxy or 1,1,2,2-tetrafluoroethyloxy; more especially by one substituent selected from unsubstituted or substituted lower alkyl, especially methyl, halo-lower alkyl, such as trifluoromethyl, unsubstituted or substituted lower alkoxy, especially methoxy and halo-lower alkoxy, especially trifluoromethoxy.

In one embodiment, the substituent is at the position which is meta or para to the bond to the pyrimidine ring.

Important R₁ phenyl radicals include unsubstituted phenyl and lower alkoxy-substituted phenyl, especially wherein the lower alkoxy substituent is at the position meta or para to the bond to the pyrimidine ring.

R₂ as a phenyl radical is preferably phenyl that is substituted in the 3-position by one substituent selected from fluorine halo-lower alkyl, especially difluoromethyl, trifluoromethyl, 2,2,2-trifluoroethyl, or 1,1,2,2-tetrafluoroethyl; halo-lower alkoxy, especially difluoromethoxy, trifluoromethoxy, 2,2,2-trifluoroethoxy, or 1,1,2,2-tetrafluoroethoxy, halo-lower alkylthio, such as difluoromethylthio, trifluoromethylthio, 2,2,2-trifluoroethylthio, or 1,1,2,2,tetrafluoroethylthio. R₂ is phenyl substituted in position 3 by one substituent selected from halo-substituted lower alkyl, halo-substituted lower alkoxy, halo-substituted lower alkylthio and fluorine.

R₂ is particularly 3-trifluoromethylphenyl.

A heteroaryl radical is especially a 5 to 7 membered aromatic ring comprising from 1 to 3 heteroatoms selected from N, O and S. The heteroaryl radical is unsubstituted or substituted by one or more, especially from one to three, for example one, identical or different substituents. Important substituents on heteroaryl radicals are those selected from the group consisting of halogen, for example, fluorine or chlorine; mono- or di-lower alkyl-substituted amino wherein the alkyl groups are unsubstituted or substituted by halogen, hydroxy, nitro, cyano, lower alkoxy, C₃-C₇ cycloalkyl, a heterocyclic radical or a heteroaryl radical; lower alkyl, such as methyl or ethyl; halo-lower alkyl, such as trifluoromethyl; lower alkoxy, such as methoxy or ethoxy; halo-lower alkoxy, for example, trifluoromethoxy; lower alkylthio, such as methylmercapto, halo-lower alkylthio, such as trifluoromethylthio, a heteroaryl radical, heteroaryl-lower-alkylene, a heterocyclic radical or heterocyclic-lower-alkylene.

Heteroaryl-lower-alkylene and heterocyclic-lower-alkylene are substituents of the formula het-C₁-C₄-alkylene- where het is a heteroaryl or heterocyclic radical.

R₁ as a heteroaryl radical is especially a thiazolyl, pyrazinyl, pyrimidinyl or 6-substituted 3-pyridyl radical, especially a 2-thiazolyl, 2-pyrazinyl, 5-pyrimidinyl radical.

Preferably, R₁ as a heteroaryl radical is particularly a 6-substituted-3-pyridyl radical.

A heterocyclic radical is especially a five- or six-membered non-aromatic ring having 1 or 2 hetero atoms selected from the group consisting of nitrogen, oxygen and sulfur, which heterocycle may be fully or partially saturated, and is unsubstituted or substituted, especially by unsubstituted or substituted lower alkyl. Heterocyclic radicals include morphilino, thiomorphilino, piperidinyl, piperazinyl, and the like.

Salts are especially the pharmaceutically acceptable acid addition salts of compounds of formula I. Such salts are formed, for example, by compounds of formula I having a basic nitrogen atom as acid addition salts, preferably with organic or inorganic acids, especially the pharmaceutically acceptable salts. Suitable inorganic acids are, for example, hydrohalic acids, such as hydrochloric acid; sulfuric acid; or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, 2-hydroxybutyric acid, gluconic acid, glucosemonocarboxylic acid, fumaric acid, succinic acid, adipic acid, pimelic acid, suberic acid, azelaic acid, malic acid, tartaric acid, citric acid, glucaric acid, galactaric acid, amino acids, such as glutamic acid, aspartic acid, N-methylglycine, acetylaminoacetic acid, N-acetylasparagine, N-acetylcysteine, pyruvic acid, acetoacetic acid, phosphoserine, 2- or 3-glycerophosphoric acid, maleic acid, hydroxymaleic acid, methylmaleic acid, cyclohexanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, glucuronic acid, galacturonic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalenedisulfonic acid, N-cyclohexylsulfamic acid, N-methyl-, N-ethyl- or N-propyl-sulfamic acid, or other organic protonic acids, such as ascorbic acid.

For isolation or purification it is also possible to use pharmaceutically unacceptable salts, for example picrates or perchlorates. Only the pharmaceutically acceptable salts or the free compounds (optionally in the form of pharmaceutical compositions) are used therapeutically, and those are therefore preferred.

In view of the close relationship between the novel compounds in free form and in the form of their salts, including also those salts which can be used as intermediates, for example in the purification of the novel compounds or for their identification, hereinbefore and hereinafter any reference to the free compounds is also to be understood as including the corresponding salts, as appropriate and expedient.

An important embodiment of the compounds of the formula I are those compounds wherein R₁ is selected from a phenyl radical, a thiazolyl radical, a pyrazinyl radical, a pyrimidinyl radical or a 6-substituted 3-pyridyl radical; especially those wherein R₂ is fluorine-phenyl (phenyl substituted by fluorine), halo-lower alkylphenyl, halo-lower alkoxyphenyl or halo-lower alkylthiophenyl; most particularly those wherein the alkyl portion of halo-lower alkylphenyl, halo-lower alkoxyphenyl or halo-lower alkylthiophenyl substituents is from mono-substituted to entirely substituted by fluorine, such as 2-fluoroethyl, trifluoromethyl or pentafluoroethyl.

Described are the compounds of formula II 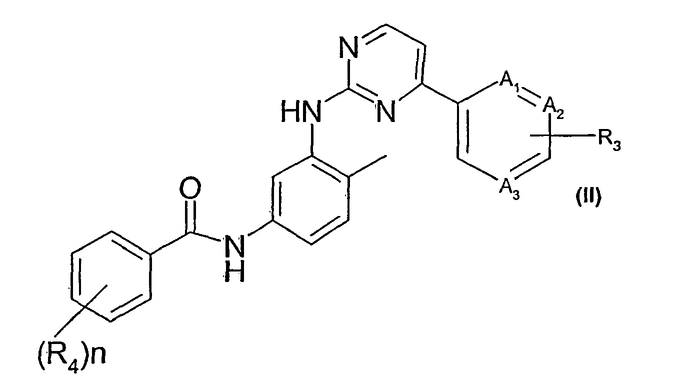 wherein
n is 0, 1 or 2;
A₁, A₂ and A₃ are C, or A₁ and A₂ are C and A₃ is N, or A₁ and A₃ are N and A₂ is C, or A₁ is C and A₂ and A₃ are N;
R₃ is -NR₅R₆, halogen, -O-R₈, -S-R₈, or lower alkyl which is unsubstituted or substituted by halogen, hydroxy, lower alkoxy, -NR₇R₈, or a heteroaryl or heterocyclic radical attached at a ring carbon;
R₄ is amino, mono- or di-lower alkyl-substituted amino, wherein the alkyl groups are unsubstituted or substituted by halogen or lower alkoxy; halogen, lower alkyl, halo-lower alkyl, lower alkoxy, halo-lower alkoxy, hydroxy, lower alkanoyl, carbamoyl, N-mono- or N,N-di-substituted carbamoyl, mercapto, lower alkylthio or halo-lower alkylthio;
R₅, R₆, R₇ and R₈ are independently hydrogen, a heteroaryl or heterocyclic radical attached at a ring carbon, lower alkyl, C₃-C₈cycloalkyl, C₃-C₈cycloalkyl-lower alkylene, lower alkyl which is substituted by hydroxy, lower alkoxy, a heteroaryl radical, a heterocyclic radical, amino, mono- or di-lower alkyl amino or R₅ and R₆ and/or R₇ and R₈ together with the nitrogen form a heteroaromatic or heterocyclic radical;
R₈ is a heterocyclic radical, a heteroaromatic radical, heteroaryl-lower-alkylene, heterocyclic-lower-alkylene, lower alkyl or lower alkyl which is substituted by hydroxy, lower alkoxy or -NR₇R₈;
or an N-oxide or a pharmaceutically acceptable salt thereof.

When R₃ is lower alkyl substituted by a heteroaryl or heterocyclic radical attached at a ring carbon, the lower alkyl is bonded to a ring carbon of the heteroaryl or heterocyclic radical, for example, 3-(4-pyridyl)propyl or 4-piperidinylmethyl.

When R₅-R₈ is a heteroaryl or heterocyclic radical attached at a ring carbon, the amino nitrogen is directly bonded to a ring carbon of the heteroaryl or heterocyclic radical, for example when -NR₅R₆ is 4-pyridylamino or 4-piperidinylamino.

At least one R₄ substituent is preferably located at the position meta to the carbonyl.

The R₄ substituents are especially selected from halogen, especially fluorine or chlorine; most particularly fluorine, mono- or di-lower alkyl-substituted amino, especially dimethylamino or diethylamino; lower alkyl, especially methyl or ethyl; halo-lower alkyl, especially difluoromethyl, trifluoromethyl, 2,2,2-trifluoroethyl, or 1,1,2,2-tetrafluoroethyl; lower alkoxy, especially methoxy or ethoxy; halo-lower alkoxy, especially difluoromethoxy, trifluoromethoxy, 2,2,2-trifluoroethoxy, and 1,1,2,2-tetrafluoroethoxy, lower alkylthio, such as methylmercapto, halo-lower alkylthio, such as difluoromethylthio, trifluoromethylthio, 2,2,2-trifluoroethylthio, or 1,1,2,2-tetrafluoroethylthio.

R₄ is especially one or two identical or different substituents selected from unsubstituted or halo-substituted lower alkyl, unsubstituted or halo-substituted lower alkoxy, unsubstituted or halo-substituted lower alkylthio and halogen, especially fluorine or chlorine.

Described are the compounds of formula (III) 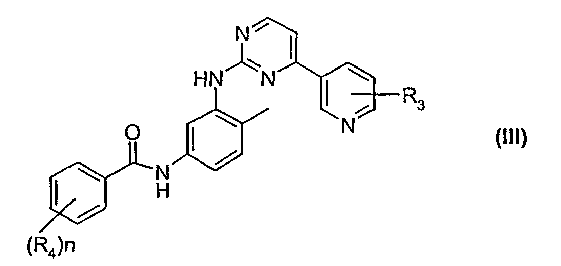 wherein
n is 0, 1 or 2;
R₃ is -NR₅R₆, halogen, -O-R₈, -S-R₈, or lower alkyl which is unsubstituted or substituted by halogen, hydroxy, lower alkoxy, -NR₇R₈, or a heteroaryl or heterocyclic radical attached at a ring carbon;
R₄ is amino, mono- or di-lower alkyl-substituted amino, wherein the alkyl groups are unsubstituted or substituted by halogen or lower alkoxy; halogen, lower alkyl, halo-lower alkyl, lower alkoxy, halo-lower alkoxy, hydroxy, lower alkanoyl, carbamoyl, N-mono- or N,N-di-substituted carbamoyl, mercapto, lower alkylthio or halo-lower alkylthio;
R₅, R₆, R₇ and R₈ are independently hydrogen, a heteroaryl or heterocyclic radical attached at a ring carbon, lower alkyl, C₃-C₈cycloalkyl, C₃-C₈cycloalkyl-lower alkylene, lower alkyl which is substituted by hydroxy, lower alkoxy, a heteroaryl radical, a heterocyclic radical, amino, mono- or di-lower alkyl amino or R₅ and R₆ or R₇ and R₈ together with the nitrogen form a heteroaromatic or heterocyclic radical;
R₈ is a heterocyclic radical, a heteroaromatic radical, heteroaryl-lower-alkylene, heterocyclic-lower-alkylene, lower alkyl or lower alkyl which is substituted by hydroxy, lower alkoxy or -NR⁷R⁸;
or an N-oxide or a pharmaceutically acceptable salt thereof.

Among the compounds of formula III, important embodiments include those compounds wherein R₄ is halo-lower alkyl, for example, trifluoromethyl, halo-lower alkoxy, for example, trifluoromethoxy, or halo-lower alkylthio, for example, trifluoromethylthio, especially those wherein one R₄ substitutent is located at the 3-position (meta) relative to the bond to the carbonyl. In an especially important embodiment, the R₄ substitutent is selected from 3-trifluoromethyl, 3-trifluoromethoxy and 3-trifluoromethylthio, especially 3-trifluoromethyl.

Further important embodiments of the compounds of formula III include those compounds wherein R₃ is -NR₅R₆ and one of R₅ and R₆ is lower alkyl substituted by -NR₇R₈ and R₇ and R₈ together with the nitrogen form a heteroaromatic or heterocyclic radical; especially wherein the heteroaromatic or heterocyclic radical is selected from morphilino, thiomorphilino, piperazinyl, piperidinyl, and pyridyl.

Further important embodiments of the compounds of formula III include those compounds wherein -NR₅R₆ is a heteroaryl or heterocyclic radical, especially piperazinyl, 4-methylpiperazinyl, piperidinyl, 4-hydroxypiperidinyl, morphilino and thiomorphilino.

Further important embodiments of the compounds of formula III include those compounds wherein R₈ is lower alkyl or lower alkyl substituted by hydroxy or lower alkoxy, especially methoxy or ethoxy; for example those compounds wherein R₃ is 2-methoxyethoxy, 3-methyoxypropyloxy, 3-ethoxypropyloxy, 3-methoxypropylthio or 2-hydroxypropylthio.

Further important embodiments of the compounds of formula III include those compounds wherein R₈ is a heteroaryl or heterocyclic radical, such as those compounds wherein R₃ is 3-pyridyloxy or 1-methyl-piperidin-4-yloxy.

In a particularly compounds of formula III, the R₃ substitutent is located at the 6-position of the pyridine ring to which it is attached. 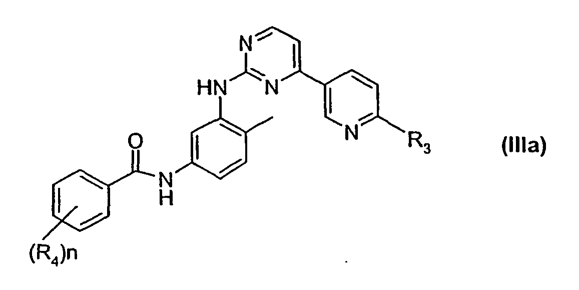 wherein the substituents and preferences are the same as given above for the compounds of formula III.

In another particularly preferred embodiment, the R₃ substitutent is located at the 6-position of the pyridine ring to which it is attached and the R₄ substitutent is attached to the 3-position of the phenyl ring. Thus, described are compounds of the formula (IIIb) 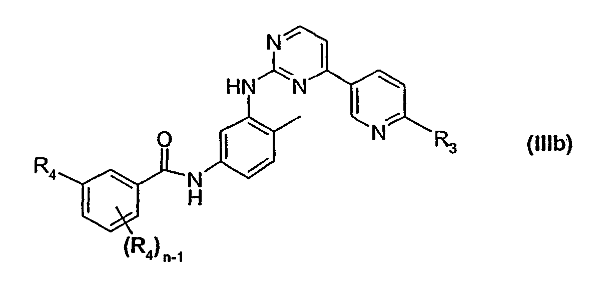 wherein the substituents and preferences are the same as given above for the compounds of formula III.

Described are compounds of formula IV 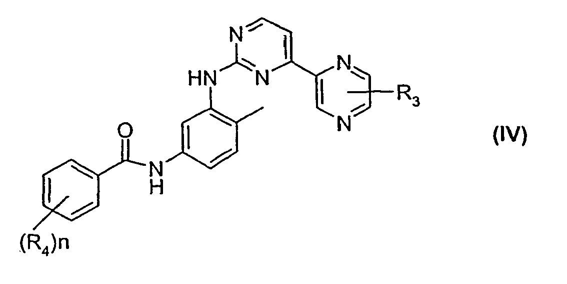 wherein
n is 0, 1 or 2;
R₃ is hydrogen, -NR₅R₆, halogen, -O-R₈, -S-R₈, or lower alkyl which is unsubstituted or substituted by halogen, hydroxy, lower alkoxy, -NR₇R₈, or a heteroaryl or heterocyclic radical attached at a ring carbon;
R₄ is amino, mono- or di-lower alkyl-substituted amino, wherein the alkyl groups are unsubstituted or substituted by halogen or lower alkoxy; halogen, lower alkyl, halo-lower alkyl, lower alkoxy, halo-lower alkoxy, hydroxy, lower alkanoyl, carbamoyl, N-mono- or N,N-di-substituted carbamoyl, mercapto, lower alkylthio or halo-lower alkylthio;
R₅, R₆, R₇ and R₈ are independently hydrogen, a heteroaryl or heterocyclic radical attached at a ring carbon, lower alkyl, C₃-C₈cycloalkyl, C₃-C₈cycloalkyl-lower alkylene, lower alkyl which is substituted by hydroxy, lower alkoxy, a heteroaryl radical, a heterocyclic radical, amino, mono- or di-lower alkyl amino or R₅ and R₆ or R₇ and R₈ together with the nitrogen form a heteroaromatic or heterocyclic radical;
R₈ is a heterocyclic radical, a heteroaromatic radical, heteroaryl-lower-alkylene, heterocyclic-lower-alkylene, lower alkyl or lower alkyl which is substituted by hydroxy, lower alkoxy or -NR⁷R⁸;
or a pharmaceutically acceptable salt thereof.

In the compounds of formula (IV), at least one R₄ substituent is preferably located at the position on the phenyl ring that is meta to the carbonyl. R₄ is preferably lower alkyl, especially methyl or ethyl; halo-lower alkyl, especially difluoromethyl, trifluoromethyl, 2,2,2-trifluoroethyl, or 1,1,2,2-tetrafluoroethyl; lower alkoxy, especially methoxy or ethoxy; halo-lower alkoxy, especially difluoromethoxy, trifluoromethoxy, 2,2,2-trifluoroethoxy, and 1,1,2,2-tetrafluoroethoxy, lower alkylthio, such as methylmercapto, halo-lower alkylthio, such as difluoromethylthio, trifluoromethylthio, 2,2,2-trifluoroethylthio, or 1,1,2,2-tetrafluoroethylthio. In an important embodiment of the compounds of formula IV, R₃ is lower alkyl, preferably lower alkyl located at the 3-position of the piperazine ring.

The compounds of the formula (V) are: 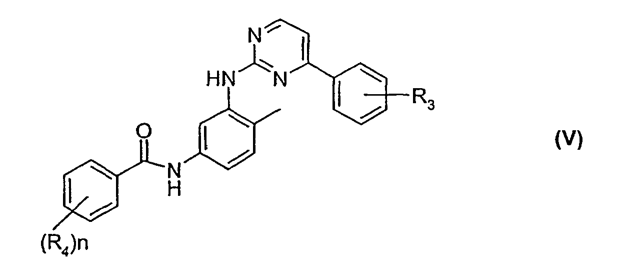 wherein
n is 0, 1 or 2;
R₃ is -NR₅R₆, halogen, -O-R₈, -S-R₈, or lower alkyl which is unsubstituted or substituted by halogen, hydroxy, lower alkoxy, -NR₇R₈, or a heteroaryl or heterocyclic radical attached at a ring carbon;
R₄ is amino, mono- or di-lower alkyl-substituted amino, wherein the alkyl groups are unsubstituted or substituted by halogen or lower alkoxy; halogen, lower alkyl, halo-lower alkyl, lower alkoxy, halo-lower alkoxy, hydroxy, lower alkanoyl, carbamoyl, N-mono- or N,N-di-substituted carbamoyl, mercapto, lower alkylthio or halo-lower alkylthio;
R₅, R₆, R₇ and R₈ are independently hydrogen, a heteroaryl or heterocyclic radical attached at a ring carbon, lower alkyl, C₃-C₈cycloalkyl, C₃-C₈cycloalkyl-lower alkylene, lower alkyl which is substituted by hydroxy, lower alkoxy, a heteroaryl radical, a heterocyclic radical, amino, mono- or di-lower alkyl amino or R₅ and R₆ or R₇ and R₈ together with the nitrogen form a heteroaromatic or heterocyclic radical;
R₈ is a heterocyclic radical, a heteroaromatic radical, heteroaryl-lower-alkylene, heterocyclic-lower-alkylene, lower alkyl or lower alkyl which is substituted by hydroxy, lower alkoxy or -NR₇R₈;
or a pharmaceutically acceptable salt thereof.

In the compounds of formula (V), at least one R₄ substituent is preferably located at the position on the phenyl ring that is meta to the carbonyl. R₄ is preferably lower alkyl, especially methyl or ethyl; halo-lower alkyl, especially difluoromethyl, trifluoromethyl, 2,2,2-trifluoroethyl, or 1,1,2,2-tetrafluoroethyl; lower alkoxy, especially methoxy or ethoxy; halo-lower alkoxy, especially difluoromethoxy, trifluoromethoxy, 2,2,2-trifluoroethoxy, and 1,1,2,2-tetrafluoroethoxy, lower alkylthio, such as methylmercapto, halo-lower alkylthio, such as difluoromethylthio, trifluoromethylthio, 2,2,2-trifluoroethylthio, or 1,1,2,2-tetrafluoroethylthio.

In an important embodiment of the compounds of formula (V), R₃ is lower alkyl or lower alkoxy, especially wherein the substituent is located meta or para to the attachment with the pyrimidine ring.

The patient is a mammal, generally a human, suffering from a disease that is characterized by excessive signaling through the MAP kinase pathway. This can be measured by activation state specific antibodies to pathway members by methods such as Western blot analysis or immunohistochemistry. Such methods are known to those of skill in the art.

In general, the disease characterized by excessive signaling through the MAP kinase signaling pathway is a proliferative disease, particularly a cancer characterized by increased RAF kinase activity, for example one which overexpresses wild-type B- or C-RAF kinase, or that expresses an activating mutant RAF kinase, for example a mutant B-RAF kinase. Cancers wherein a mutated RAF kinase has been detected include melanoma, colorectal cancer, ovarian cancer, gliomas, adenocarcinomas, sarcomas, breast cancer and liver cancer. Mutated B-RAF kinase is especially prevalent in many melanomas.

In accordance with the present invention, a sample of diseased tissue may taken from the patient, for example, as a result of a biopsy or resection, and tested to determine whether the tissue produces a mutant RAF kinase, such as a mutant B-RAF kinase or overexpresses a wild-type RAF kinase, such as wild-type B- or C-RAF kinase. If the test indicates that mutant RAF kinase is produced or that a RAF kinase is overproduced in the diseased tissue, the patient is treated by administration of an effective RAF-inhibiting amount of a RAF inhibitor compound described herein.

However, it is also possible to downregulate the MAP kinase signaling pathway with a RAF kinase inhibiting compound if another kinase in the cascade is the cause of excessive signaling in the pathway. Thus, the present invention further relates to the treatment of a disease characterized by excessive signaling in the MAP kinase signaling pathway attributed to a cause other than an activating mutation in or overexpression of a RAF kinase.

Tissue samples are tested by methods generally known in the art. For example, B-RAF mutations are detected by allele specific PCR, DHPLC, mass spectropscopy and overexpression of wild-type B- or C-RAF detected by immunohistochemistry, immunofluorescense, or Western blot analysis. A particularly useful method of detecting B-RAF mutations is the polymerase chain reaction based method described in Example D1. Similar methods are used to determine whether other kinases in the cascade are mutant or overexpressed.

Generally, the B-RAF kinase mutation is one of those described in the Davies et al article cited. These mutations are summarized in Table 1.

**Table 1**

| B-RAF mutation | protein change |
|---|---|
| G1388A | G463E |
| G1388T | G463V |
| G1394C | G465A |
| G1394A | G465E |
| G1394T | G465V |
| G1403C | G468A |
| G1403A | G468E |
| G1753A | E585K |
| T1782G | F594L |
| G1783C | G595R |
| C1786G | L596V |
| T1787G | L596R |
| T1796A | V599E |
| TG1796-97AT | V599D |

Thus, the present invention particularly relates to compounds of formula (I), useful as medicaments for treating a disease characterized by an activated mutant B-RAF kinase, which comprises detecting a mutation in the B-RAF kinase gene or protein in a tissue sample from a patient and use of a compound of formula I for preparing a medicament for treating the patient.

An important aspect of this invention includes those instances wherein the mutant B-RAF kinase exhibits a mutation described in Table 1, especially the V599E mutation.

A particularly important aspect of this invention includes those instances wherein disease is melanoma and the mutant B-RAF kinase exhibits a mutation described in Table 1, especially the V599E mutation.

Accordingly, this invention includes compounds for treating a disease characterized by mutant B-RAF kinase, which comprises detecting a mutation in the B-RAF kinase gene selected from G1388A, G1388T, G1394C, G1394A, G1394T, G1403C, G1403A, G1753A, T1782G, G1783C, C1786G, T1787G, T1796A and TG1796-97AT, or corresponding mutation in the RAF kinase protein, in a tissue sample from a patient and treating the patient with an effective B-RAF kinase inhibiting compound described herein.

The present invention further relates to compounds for inhibiting RAF kinase, which comprises contacting the RAF kinase with a compound of formula (I), (II), (III), (IV) or (V). Preferably, the RAF kinase is B- or C-RAF kinase, or a mutant RAF kinase, especially a mutant B-RAF kinase, particularly the V599E mutant. The RAF kinase may be isolated or in a cellular environment.

The compounds of formula I (or N-oxides thereof) have valuable pharmacological properties, as described above.

The compounds of the present invention may be administered alone or in combination with other anticancer agents, such as compounds that inhibit tumor angiogenesis, for example, the protease inhibitors, epidermal growth factor receptor kinase inhibitors, vascular endothelial growth factor receptor kinase inhibitors and the like; cytotoxic drugs, such as antimetabolites, like purine and pyrimidine analog antimetabolites; antimitotic agents like microtubule stabilizing drugs and antimitotic alkaloids; platinum coordination complexes; antitumor antibiotics; alkylating agents, such as nitrogen mustards and nitrosoureas; endocrine agents, such as adrenocorticosteroids, androgens, anti-androgens, estrogens, antiestrogens, aromatase inhibitors, gonadotropin-releasing hormone agonists and somatostatin analogues and compounds that target an enzyme or receptor that is overexpressed and/or otherwise involved a specific metabolic pathway that is upregulated in the tumor cell, for example ATP and GTP phosphodiesterase inhibitors, protein kinase inhibitors, such as serine, threonine and tyrosine kinase inhibitors, for example, Abelson protein tryosine kinase and the various growth factors, their receptors and kinase inhibitors therefore, such as, epidermal growth factor receptor kinase inhibitors, vascular endothelial growth factor receptor kinase inhibitors, fibroblast growth factor inhibitors, insulin-like growth factor receptor inhibitors and platelet-derived growth factor receptor kinase inhibitors and the like; methionine aminopeptidase inhibitors, proteasome inhibitors, cyclooxygenase inhibitors, for example, cyclooxygenase-1 or -2 inhibitors, and histone deacetylase inhibitors.

The compound of the present invention may also be administered together with radiotherapy, immunotherapy, surgical treatment or combinations thereof. Treatment to maintain the status of a patient after tumor remission or even chemopreventive treatment, for example in the case of at-risk patients, is also possible.

Compounds according to the invention are intended not only for the (prophylactic and, preferably, therapeutic) treatment of human beings, but also for the treatment of other warm-blooded animals, for example of commercially useful animals, for example rodents, such as mice, rabbits or rats, or guinea pigs.

In general, the invention relates also to the use of a compound of formula I (or an N-oxide thereof) in inhibiting RAF kinase activity.

The compounds of the present invention are preferably administered as an active ingredient in a pharmaceutical composition. Preference is given to a pharmaceutical composition which is suitable for administration to a warm-blooded animal, especially a human being or a commercially useful mammal, which is suffering from a disease characterized by an aberrant MAP kinase signaling pathway especially, a tumor disease, most particularly melanoma, comprising a compound of formula I (or an N-oxide thereof), or a pharmaceutically acceptable salt thereof where salt-forming groups are present, in an amount that is effective in inhibiting RAF kinase, particularly a mutant RAF kinase, together with at least one pharmaceutically acceptable carrier.

Preference is given also to a pharmaceutical composition for the prophylactic or, especially, therapeutic treatment of tumor diseases and other proliferative diseases in a warm-blooded animal, especially a human being or a commercially useful mammal, which requires such treatment, especially which is suffering from such a disease, comprising a novel compound of formula I (or an N-oxide thereof), or a pharmaceutically acceptable salt thereof, as active ingredient in an amount that is effective prophylactically or, especially, therapeutically against the mentioned diseases.

Pharmaceutical compositions comprise from approximately 1 % to approximately 95 % active ingredient, dosage forms that are in single dose form preferably comprising from approximately 20 % to approximately 90 % active ingredient, and dosage forms that are not in single dose form preferably comprising from approximately 5 % to approximately 20 % active ingredient. Unit dose forms are, for example, dragées, tablets, ampoules, vials, suppositories or capsules. Other dosage forms are, for example, ointments, creams, pastes, foams, tinctures, lipsticks, drops, sprays, dispersions, etc.. Examples are capsules comprising from approximately 0.05 g to approximately 1.0 g of the active ingredient.

The pharmaceutical compositions of the present invention are prepared in a manner known per se, for example by means of conventional mixing, granulating, confectioning, dissolving or lyophilising processes.

Solutions of the active ingredient are preferably used, in addition also suspensions or dispersions, especially isotonic aqueous solutions, dispersions or suspensions, which, in the case of, for example, lyophilised compositions which contain the active substance alone or together with a carrier, for example mannitol, can be prepared prior to use. The pharmaceutical compositions may be sterilised and/or comprise excipients, for example preservatives, stabilisers, wetting agents and/or emulsifiers, solubilisers, salts for regulating the osmotic pressure and/or buffers, and are prepared in a manner known per se, for example by means of conventional dissolving or lyophilising processes. The mentioned solutions or suspensions may comprise viscosity-increasing substances, such as sodium carboxymethylcellulose, carboxymethylcellulose, dextran, polyvinylpyrrolidone or gelatin, or solubilisers, for example Tween 80 [polyoxyethylene(20)sorbitan monooleate; trade mark of ICI Americas, Inc, USA].

Suspensions in oil comprise as the oily component the vegetable, synthetic or semi-synthetic oils customary for injection purposes. There may be mentioned as such especially liquid fatty acid esters, which comprise as the acid component a long-chained fatty acid having from 8 to 22, especially from 12 to 22, carbon atoms, for example lauric acid, tridecylic acid, myristic acid, pentadecylic acid, palmitic acid, margaric acid, stearic acid, arachidic acid, behenic acid or corresponding unsaturated acids, for example oleic acid, elaidic acid, erucic acid, brassidic acid or linoleic acid, optionally with the addition of antioxidants, for example vitamin E, β-carotene or 3,5-di-tert-butyl-4-hydroxytoluene. The alcohol component of those fatty acid esters has a maximum of 6 carbon atoms and is a mono- or poly-hydric, for example mono-, di- or tri-hydric, alcohol, for example methanol, ethanol, propanol, butanol or pentanol or their isomers, but especially glycol and glycerol. Examples of fatty acid esters which may be mentioned are, therefore: ethyl oleate, isopropyl myristate, isopropyl palmitate, "Labrafil M 2375" (polyoxyethyleneglycerol trioleate from Gattefossé, Paris), "Labrafil M 1944 CS" (unsaturated polyglycolised glycerides prepared by alcoholysis of apricot kernel oil and composed of glycerides and polyethylene glycol ester; Gattefossé, France), "Labrasol" (saturated polyglycolised glycerides prepared by alcoholysis of TCM and composed of glycerides and polyethylene glycol ester; Gattefossé, France) and/or "Miglyol 812" (triglyceride of saturated fatty acids having a chain length of from C₈ to C₁₂ from Hüls AG, Germany), but especially vegetable oils, such as cottonseed oil, almond oil, olive oil, castor oil, sesame oil, soybean oil and, more especially, groundnut oil.

The preparation of the injection compositions is carried out in customary manner under sterile conditions, as are also the introduction thereof, for example, into ampoules or vials and the sealing of the containers.

Pharmaceutical compositions for oral administration can be obtained, for example, by combining the active ingredient with one or more solid carriers, granulating a resulting mixture, where appropriate, and processing the mixture or granules, if desired, where appropriate by addition of additional excipients, to tablets or dragée cores.

Suitable carriers are especially fillers, such as sugars, for example lactose, saccharose, mannitol or sorbitol, cellulose preparations and/or calcium phosphates, for example tricalcium phosphate or calcium hydrogen phosphate, also binders, such as starches, for example corn, wheat, rice or potato starch, methylcellulose, hydroxypropylmethylcellulose, sodium carboxymethylcellulose and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Dragée cores can be provided with suitable, optionally enteric, coatings, there being used inter alia concentrated sugar solutions which may contain gum arabic, talc, polyvinylpyrrolidone, polyethylene glycol and/or titanium dioxide, or coating solutions in suitable organic solvents or solvent mixtures or, for the preparation of enteric coatings, solutions of suitable cellulose preparations, such as acetylcellulose phthalate or hydroxypropylmethylcellulose phthalate. Colourings or pigments may be added to the tablets or dragée coatings, for example for identification purposes or to indicate different doses of active ingredient.

Pharmaceutical compositions for oral administration are also hard gelatin capsules and soft sealed capsules consisting of gelatin and a plasticiser, such as glycerol or sorbitol. The hard gelatin capsules may contain the active ingredient in the form of granules, for example in admixture with fillers, such as corn starch, binders and/or glidants, such as talc or magnesium stearate, and optionally stabilisers. In soft capsules the active ingredient is preferably dissolved or suspended in suitable liquid excipients, such as fatty oils, paraffin oil or liquid polyethylene glycols or fatty acid esters of ethylene glycol or propylene glycol, it likewise being possible to add stabilisers and detergents, for example of the polyoxyethylenesorbitan fatty acid ester type.

Suitable rectally administrable pharmaceutical compositions are, for example, suppositories that consist of a combination of the active ingredient with a suppository base. Suitable suppository bases are, for example, natural or synthetic triglycerides, paraffin hydrocarbons, polyethylene glycols or higher alkanols.

For parenteral administration there are suitable, especially, aqueous solutions of an active ingredient in water-soluble form, for example in the form of a water-soluble salt, or aqueous injection suspensions that comprise viscosity-increasing substances, for example sodium carboxymethylcellulose, sorbitol and/or dextran and, if desired, stabilisers. The active ingredient, optionally together with excipients, can also be in the form of a lyophilisate and can be made into a solution prior to parenteral administration by the addition of suitable solvents.

Solutions used, for example, for parenteral administration can also be used as infusion solutions.

Preferred preservatives are, for example, antioxidants, such as ascorbic acid, or microbicides, such sorbic acid or benzoic acid.

The invention relates especially to compounds for treating one of the pathological conditions that is characterized by an aberrant MAP kinase signaling pathway, especially a disease responsive to inhibition of RAF kinase, especially a corresponding tumor disease. The compounds of formula I (or an N-oxide thereof) can be administered prophylactically or therapeutically as such or in the form of pharmaceutical compositions, preferably in an amount that is effective against the mentioned diseases, to a warm-blooded animal, for example a human being, requiring such treatment, the compounds being used especially in the form of pharmaceutical compositions. In the case of a body weight of approximately 70 kg, a daily dose of from approximately 0.1 g to approximately 5 g, preferably from approximately 0.5 g to approximately 2 g, of a compound of the present invention is administered.

The preferred dosage, composition and preparation of pharmaceutical formulations (medicaments) to be used in each particular case are described above.

The compounds of the present invention are prepared utilizing methods known to those of ordinary skill in the art according to the general reaction schemes described below..

### Step 1

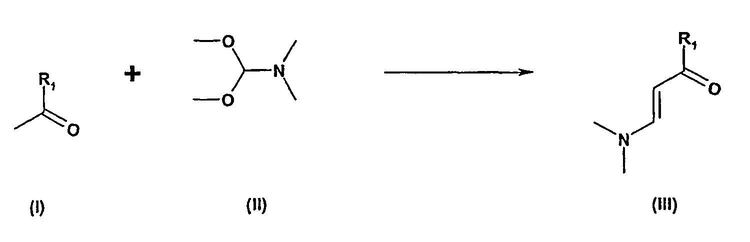

A mixture of I and II are heated to 85 °C. After stirring overnight, the mixture is cooled first in air then in an ice/water bath and filtered. The resulting solid is washed well with ether and dried to yield III

### Preparation of common intermediate VII

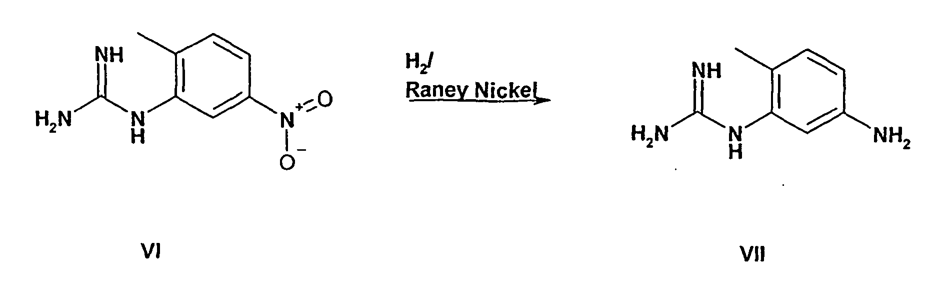

Raney nickel is charged to a degassed solution of VI in methanol (100 mL) and covered with hydrogen(60 psi.). The mixture is shaken overnight and filtered. Concentration in vacuo yields VII as a brown solid.

### Reaction of III and VII

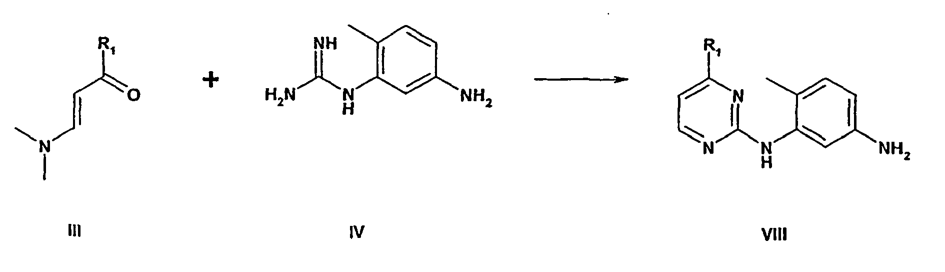

III and IV are dissolved in 2-propanol with heating at about 85°C. After stirring for 24 hours, the mixture is cooled and filtered. The resulting solid is washed well with ether and dried to yield V.

### Final Step

V is reacted with an appropriately substituted benzoic acid or benzoic acid halide under standard conditions to yield the final benzamide.

Compounds of formula VII and VIII are described as intermediates for the preparation of the compounds of formulae (I), II, II, III, III(a), III(b) IV and V.

### Alternative Synthetic Scheme

The alternative synthetic scheme discloses the synthesis of a common intermediate for the preparation of compounds of formula III wherein R₄ is 3-trifluoromethyl. One of skill in the art has the ability to substitute various starting materials in order to prepare the inventive compounds.

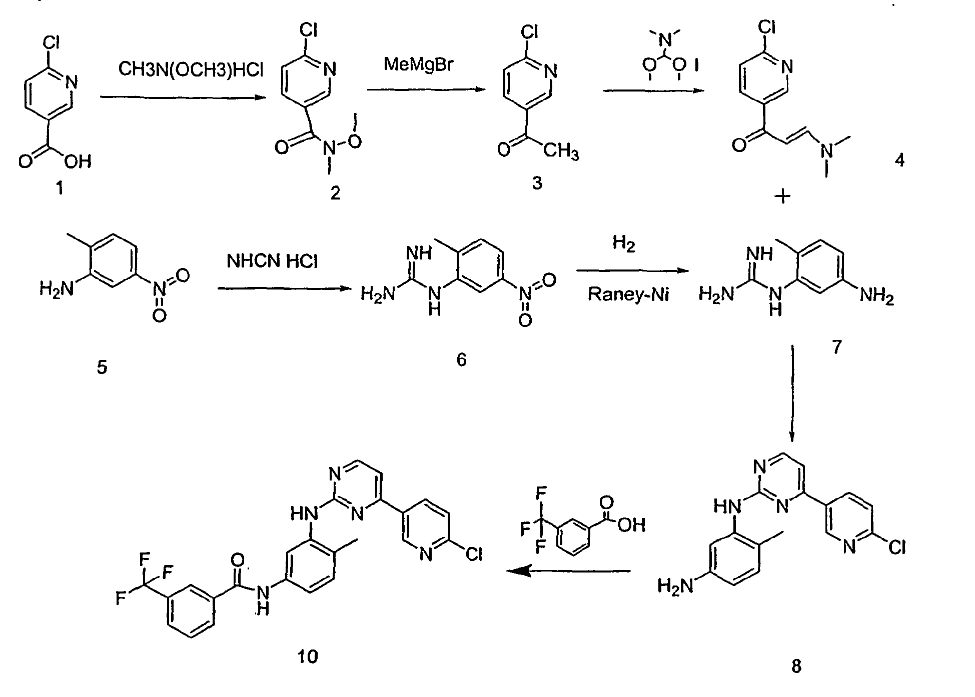

Compound of formula 8 is an intermediate for the preparation of the compounds of formula III, III(a) and III(b).

The Examples which follow serve to illustrate the invention without limiting the scope thereof.

### Example 1

### N-[4-Methyl-3-(4-thiazol-2-yl-pyrimidin-2-ylamino)-phenyl-3-trifluoromethyl-benzamide

A mixture of 4-Methyl-N*3*-(4-thiazol-2-yl-pyrimidin-2-yl)-benzene-1,3-diamine (0.17g, 0.60 mmol), 3-Trifluoromethyl-benzoic acid (0.12g, 0.63 mmol), BOP (0.32g, 0.72 mmol) and DIEA (0.4 mL, 2.16 mmol) is stirred in 2.5 mL of DMF at room temperature, under nitrogen overnight. After 17.5 hours of stirring, 20 mL of deionized water is added to the precipitate. The product and the mixture are allowed to stir at room temperature for 45 minutes. They are then filtered through a Buchner funnel, triturated with 10.0 mL of CH₃OH, stirred for 15 minutes, filtered again, rinsed with 10.0 mL of CH₃OH and dried. 0.11 g of N-[4-Methyl-3-(4-thiazol-2-yl-pyrimidin-2-ylamino)-phenyl]-3-trifluoromethyl-benzamide is obtained as a yellow solid.

¹H NMR (DMSO, 500MHz): δ (ppm) 10.41 (s, 1H), 9.17 (s, 1H), 8.64 (d, 1H, J=5.05 Hz), 8.29 (br m, 1H), 8.26 (d, 1H, J=7.88 Hz), 8.06 (d, 1H, J=3.15 Hz), 7.96 (br m, 1H), 7.94 (d, 1H, J=3.16 Hz), 7.91 (1H, d, J=2.27 Hz), 7.77 (t, 1H, J=7.88 Hz), 7.56 (dd, 1H, J₁=8.35 Hz, J₂=2.45 Hz), 7.39 (d, 1H, J=4.39 Hz), 7.24 (d, 1H, J=8.79 Hz), 2.22 (s, 3H).

API-MS m/z 456.1 ([M+H]⁺, calcd 456.1)

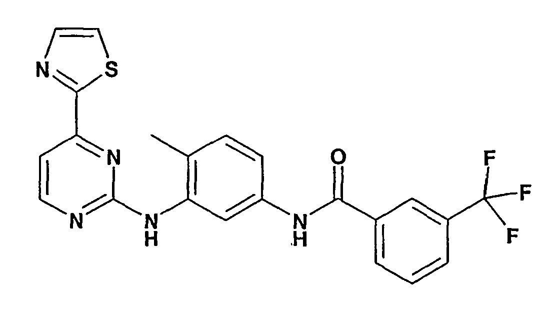

### Example 2

### N-[4-Methyl-3-(4-pyrazin-2-yl-pyrimidin-2-ylamino)-phenyl]-3-trifluoromethyl-benzamide

A mixture of 4-Methyl-N*3*-(4-pyrazin-2-yl-pyrimidin-2-yl)-benzene-1,3-diamine (0.36g, 1.30 mmol), 3-Trifluoromethyl-benzoic acid (0.26g, 1.36 mmol), BOP (0.69g, 1.56 mmol) and DIEA (0.8 mL, 4.68 mmol) is stirred in 5.0 mL of DMF at room temperature, under Nitrogen overnight. 50 mL of deionized water is added to the resulting precipitate. The mixture is allowed to stir at room temperature for 45 minutes and then is filtered through a Buchner funnel, triturated with 25.0 mL of CH₃OH, stirred for 15 minutes, filtered again, rinsed with 25.0 mL of CH₃OH and dried. 0.41 g of N-[4-Methyl-3-(4-pyrazin-2-yl-pyrimidin-2-ylamino)-phenyl]-3-trifluoromethyl-benzamide is obtained as a pale yellow solid.

¹H NMR (DMSO, 500MHz): δ (ppm) 10.45 (s, 1H), 9.49 (s,1H), 9.10 (s,1H), 8.79 (s, 2H), 8.63 (d, 1H, J=5.39 Hz), 8.31 (br m, 1H), 8.28 (d, 1H, J=8.08 Hz), 8.16 (d, 1H, J=2.37 Hz), 7.96 (d, 1H, J=8.08 Hz), 7.79 (t, 1H, J=8.08 Hz), 7.63 (d, 1H, J=4.87 Hz), 7.50 (dd, 1H, J₁=8.67 Hz, J₂=2.17 Hz), 7.26 (d, 1H, J=7.59 Hz), 2.26 (s, 3H).

API-MS m/z 451.1 ([M+H]⁺, calcd 451.1)

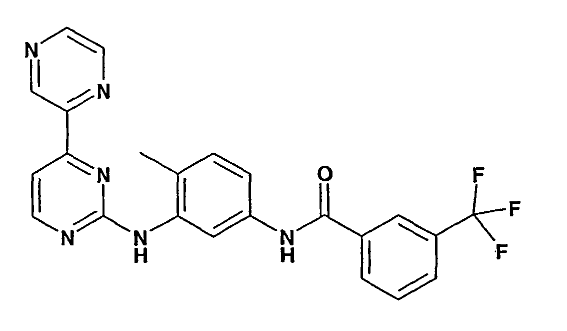

### Example 3

### N-[4-Methyl-3-(4-pyrazin-2-yl-pyrimidin-2-ylamino)-phenyl]-3-(1,1,2,2-tetrafluoro-ethoxy)-benzamide

A mixture of 4-Methyl-N*3*-(4-pyrazin-2-yl-pyrimidin-2-yl)-benzene-1,3-diamine (0.55 g, 1.98 mmol), 3-(1,1,2,2-Tetrafluoro-ethoxy)-benzoic acid (0.50 g, 2.08 mmol), BOP (1.05 g, 2.38 mmol) and DIEA (1.25 mL, 7.14 mmol) is stirred in 10.0 mL of DMF at room temperature for 20 hours. 50 mL of deionized water is added to the resulting precipitate. The resulting suspension is allowed to stir at room temperature for 45 minutes. The mixture is then filtered through a Buchner funnel, triturated with 20.0 mL of CH₃OH, stirred for 15 minutes, filtered again, rinsed with 20.0 mL of CH₃OH and dried. 0.71 g of N-[4-Methyl-3-(4-pyrazin-2-yl-pyrimidin-2-ylamino)-phenyl]-3-(1,1,2,2-tetrafluoro-ethoxy)-benzamide is obtained as a pale, yellow solid.

¹H NMR (DMSO, 500MHz): δ (ppm) 10.36 (s, 1H), 9.49 (s, 1H), 9.10 (s, 1H), 8.79 (s,2H), 8.62 (d, 2H, J=5.13 Hz), 8.15 (br m, 1H), 7.99 (d, 2H, J=8.33 Hz), 7.86 (br m, 1H), 7.65(m, 2H), 7.51 (m, 2H), 7.25 (d, 1H,J=8.4 Hz), 6.86 (t,1H, J=52.4Hz), 2.26 (s, 3H).

API-MS m/z 499.4 ([M+H]⁺, calcd 499.4)

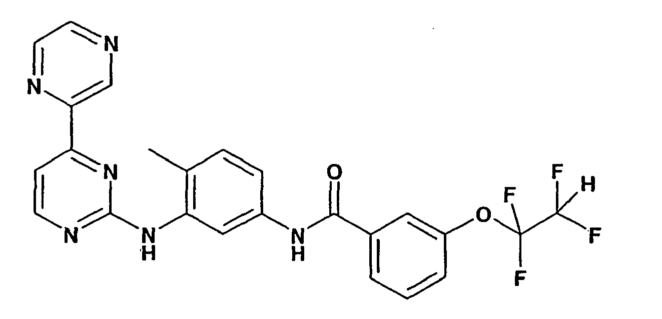

### Example 4

### 3-Difluoromethoxy-N-[4-methyl-3-(4-pyrazin-2-yl-pyrimidin-2-ylamino)-phenyl]-benzamide

A mixture of 4-Methyl-N*3*-(4-pyrazin-2-yl-pyrimidin-2-yl)-benzene-1,3-diamine (0.55 g, 1.98 mmol), 3-(2,2-Difluoro-ethoxy)-benzoic acid (0.39 g, 2.08 mmol), BOP (1.05 g, 2.38 mmol) and DIEA (1.25 mL, 7.14 mmol) is stirred in 10.0 mL of DMF at room temperature overnight. 50 mL of deionized water is added to the precipitate. The resulting suspension is allowed to stir at room temperature for 45 minutes. It is then filtered through a Buchner funnel, triturated with 20.0 mL of CH₃OH, stirred for 15 minutes, filtered again, rinsed with 20.0 mL of CH₃OH and dried. 0.79 g of 3-3-Difluoromethoxy-N-[4-methyl-3-(4-pyrazin-2-yl-pyrimidin-2-ylamino)-phenyl]-benzamide is obtained as a pale yellow solid.

¹H NMR (DMSO, 500MHz): δ (ppm) 10.29 (s, 1H), 9.47 (s, 1H), 9.09 (s, 1H), 8.79 (s,1H), 8.61 (d, 1H, J₁=5.45 Hz, J₂=1.82 Hz), 8.14 (m, 1H), 7.85 (d, 1H, J=7.61 Hz), 7.64-7.56 (m, 2H), 7.51-7.46 (m, 1H), 7.40(dd, 1H, J₁=7.62 Hz, J₂=2.77 Hz), 7.33 (t, 1H, J=74.2 Hz), 7.24 (d, 1H, J=8.32 Hz), 2.25(s, 3H).

API-MS m/z 449.3 ([M+H]⁺, calcd 449.4)

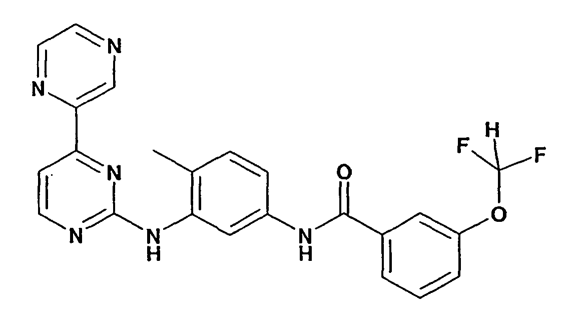

### Example 5

### 3-Dimethylamino-N-[4-methyl-3-(4-pyrazin-2-yl-pyrimidin-2-ylamino)-phenyl]-benzamide

A mixture of 4-Methyl-N*3*-(4-pyrazin-2-yl-pyrimidin-2-yl)-benzene-1,3-diamine (0.55 g, 1.98 mmol), 3-Dimethylamino-benzoic acid (0.34 g, 2.08 mmol), BOP (1.05 g, 2.38 mmol) and DIEA (1.25 mL, 7.14 mmol) is stirred in 10.0 mL of DMF at room temperature for 20 hours. 50 mL of deionized water is added to the precipitate. The resulting suspension is allowed to stir at room temperature for 45 minutes. It is then filtered through a Buchner funnel, triturated with 20.0 mL of CH₃OH, stirred for 15 minutes, filtered again, rinsed with 20.0 mL of CH₃OH and dried. 0.64 g of 3-Dimethylamino-N-[4-methyl-3-(4-pyrazin-2-yl-pyrimidin-2-ylamino)-phenyl]-benzamide is obtained as an off-white solid.

¹H NMR (DMSO, 500MHz): δ (ppm) 10.09 (s, 1H), 9.47 (s, 1H), 9.07 (s, 1H), 8.79 (s,1H), 8.61 (d, 1H, J =5.45 Hz,), 8.14 (m, 1H), 7.62 (d, 1H, J=5.52 Hz), 7.50 (dd, 1H, J₁=8.47 Hz, J₂=2.31 Hz), 7.31 (dd, 1H, J=8.08 Hz, J=8.08 Hz), 7.24 (m,2H), 7.21 (m, 1H),6.92 (dd, 1H, J₁=8.48 Hz, J₂=2.31 Hz), 2.97 (s, 6H), 2.25 (s, 3H).

API-MS m/z 426.1 ([M+H]⁺, calcd 426.2)

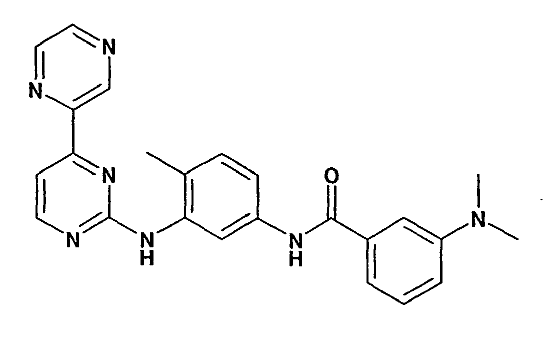

### Example 6

### N-[4-Methyl-3-(4-pyrazin-2-yl-pyrimidin-2-ylamino)-phenyl]-3-(2,2,2-trifluoro-ethoxy)-benzamide

A mixture of 4-Methyl-N*3*-(4-pyrazin-2-yl-pyrimidin-2-yl)-benzene-1,3-diamine (0.55 g, 1.98 mmol), 3-(2,2,2-Trifluoro-ethoxy)-benzoic acid (0.46 g, 2.08 mmol), BOP (1.05 g, 2.38 mmol) and DIEA (1.25 mL, 7.14 mmol) is stirred in 10.0 mL of DMF at room temperature for 20 hours. 50 mL of deionized water is added to the precipitate. The resulting suspension is allowed to stir at room temperature for 45 minutes. It is then filtered through a Buchner funnel, triturated with 25.0 mL of CH₃OH, stirred for 15 minutes, filtered again, rinsed with 25.0 mL of CH₃OH and dried. 0.63 g of N-[4-Methyl-3-(4-pyrazin-2-yl-pyrimidin-2-ylamino)-phenyl]-3-(2,2,2-trifluoro-ethoxy)-benzamide is obtained as a light yellow solid.

¹H NMR (DMSO, 300MHz): δ (ppm) 10.21 (s, 1H), 9.48 (s, 1H), 9.10 (s, 1H), 8.79 (s,2H), 8.62 (d, 1H, J=4.82 Hz), 8.15 (br m, 1H), 7.63 (m, 3H), 7.50 (m, 2H), 7.29 (dd, 1H, J₁=8.05 Hz, J₂=2.15 Hz), 7.24 (d, 1H, J=8.58 Hz), 4.86(q, 2H,J=8.8 Hz), 2.25 (s, 3H).

API-MS m/z 481.3 ([M+H]⁺, calcd 481.1)

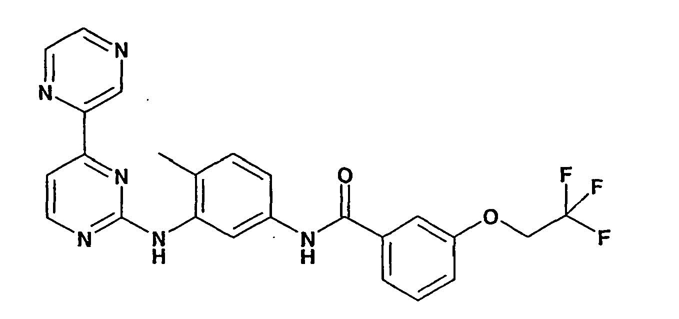

### Example 7

### N-[4-Methyl-3-(4-pyrazin-2-yl-pyrimidin-2-ylamino)-phenyl]-3-trifluoromethylsulfanyl-benzamide

A mixture of 4-Methyl-N*3*-(4-pyrazin-2-yl-pyrimidin-2-yl)-benzene-1,3-diamine (0.55 g, 1.98 mmol), 3-Trifluoromethylsulfanyl-benzoic acid (0.46 g, 2.08 mmol), BOP (1.05 g, 2.38 mmol) and DIEA (1.25 mL, 7.20 mmol) is stirred in 6.0 mL of DMF at room temperature overnight. 50 mL of water is added to the precipitate. The product and the mixture are allowed to stir at room temperature for one hour and then are filtered through a Buchner funnel, air dried and triturated with 10.0 mL of CH₃OH for 45 minutes and dried. 0.78 g of N-[4-Methyl-3-(4-pyrazin-2-yl-pyrimidin-2-ylamino)-phenyl]-3-trifluoromethylsulfanyl-benzamide is obtained as a yellow solid.

¹H NMR (DMSO, 500MHz): δ (ppm) 10.40 (s,1H), 9.48 (s, 1H), 9.10 (s, 1H), 8.79(s, 2H), 8.62 (d, 1H, J=5.38 Hz), 8.30 (br m, 1H), 8.20(d, 1H, J=7.68 Hz), 8.15 (m,1H), 7.94(d, 1H, J=7.68 Hz), 7.72 (t, 1H, J=8.06 Hz), 7.63 (d, 1H, J=5.38 Hz), 7.50 (dd, 1H, J₁=8.44 Hz, J₂=2.29 Hz), 7.25 (d, 1H, J=8.44 Hz), 2.26 (s, 3H).

API-MS m/z 483.1 ([M+H]⁺, calcd 483.1)

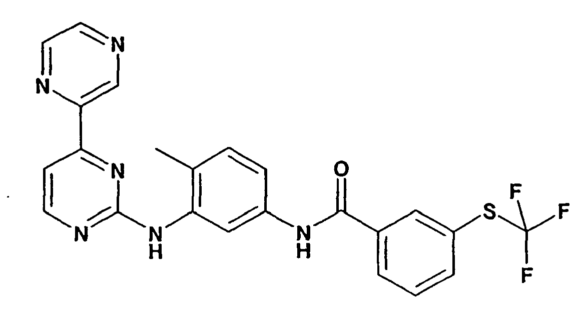

### Example 8

### N-{3-[4-(4-Methoxy-phenyl)-pyrimidin-2-ylamino]-4-methyl-phenyl}-3-trifluoromethyl-benzamide

A mixture of N*3*-[4-(4-Methoxy-phenyl)-pyrimidin-2-yl]-4-methyl-benzene-1,3-diamine (0.29g, 0.95 mmol), 3-Trifluoromethyl-benzoic acid (0.19g, 0.99 mmol), BOP (0.50g, 1.14 mmol) and DIEA (0.6 mL, 3.42 mmol) is stirred in 5.0 mL of DMF at room temperature overnight. 50 mL of water is added to the precipitate. The product and the mixture are allowed to stir at room temperature for one hour then are filtered through a Buchner funnel, air dried and triturated with 6.0 mL of CH₃OH for 45 minutes and dried. 0.38 g of N-{3-[4-(4-Methoxy-phenyl)-pyrimidin-2-ylamino]-4-methyl-phenyl}-3-trifluoromethyl-benzamide is obtained as a yellow solid.

¹H NMR (DMSO, 500MHz): δ (ppm) 10.43 (s, 1H), 8.40 (d, 2H, J=5.21Hz), 8.31 (s, 1H), 8.28(d, 1H, J=7.89Hz), 8.13 (s, 1H), 8.11 (m, 2H, br),7.96(d, 1H, J=8.35Hz), 7.79(t, 1H,J=7.72Hz), 7.50 (d, 1H, J=8.20Hz), 7.28(d, 1H, J=5.21 Hz), 7.23(d, 1H, J=8.35Hz), 7.02 (d, 1H, J=8.99Hz), 3.81 (s, 3H),2.26 (s, 3H).

API-MS m/z 479.2 ([M+H]⁺, calcd 479.2)

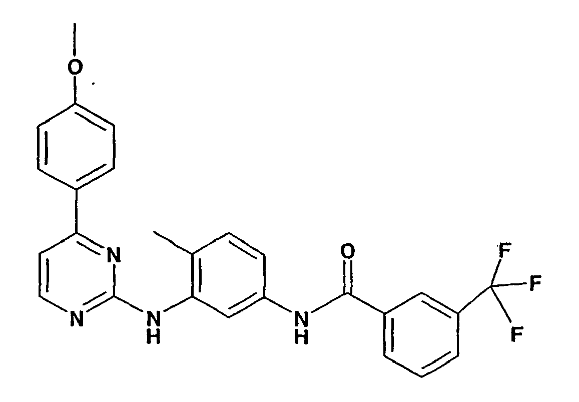

### Example 9

### N-[4-Methyl-3-(4-phenyl-pyrimidin-2-ylamino)-phenyl]-3-trifluoromethyl-benzamide

A mixture of 4-Methyl=N*3*-(4-phenyl-pyrimidin-2-yl)-benzene-1,3-diamine (0.50g, 1.81 mmol), 3-Trifluoromethyl-benzoic acid (0.36g, 1.90 mmol), BOP (0.96g, 2.17 mmol) and DIEA (1.25 mL, 6.51 mmol) is stirred in 6.0 mL of DMF at room temperature overnight. 50 mL of water is added to the precipitate. The product and the mixture are allowed to stir at room temperature for one hour, then are filtered through a Buchner funnel, air dried and triturated with 10.0 mL of CH₃OH for 45 minutes and dried. 0.27 g of a yellow solid is obtained. This product is again triturated with 5.0 mL of CH₃OH for 1 hour, filtered and dried. 0.12 g of N-[4-Methyl-3-(4-phenyl-pyrimidin-2-ylamino)-phenyl]-3-trifluoromethyl-benzamide is obtained as a pale yellow solid.

¹H NMR (DMSO, 500MHz): δ (ppm) 10.43 (s, 1H), 8.90 (s, 1H), 8.48 (d, 1H,J=4.94Hz), 8.30( m,1H), 8.27 (d, 1H, J=7.40 Hz), 8.13-8.16 (m, 2H), 8.09 (d, 1H, J=2.19), 7.97 (d, 1H, J=7.11Hz), 7.79(t, 1H,J=7.66Hz), 7.48- 7.52(m, 2H), 7.36 (d, 1H, J=5.47 Hz), 7.24 (d, 1H, J=8.20 Hz), 2.26 (s, 3H).

API-MS m/z 449.3 ([M+H]⁺, calcd 449.4)

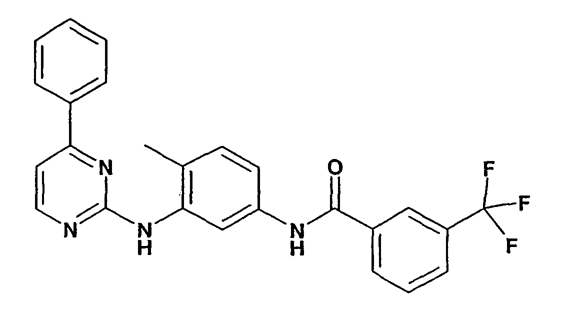

### Example 10

### N-{3-[4-(3-Methoxy-phenyl)-pyrimidin-2-ylamino]-methyl-phenyl}-3-trifluoromethyl-benzamide

A mixture of N*3*-[4-(3-Methoxy-phenyl)-pyrimidin-2-yl}-4-methyl-benzene-1,3-diamine (0.40g, 1.30 mmol), 3-Trifluoromethyl-benzoic acid (0.26g, 1.36 mmol), BOP (0.69g, 1.56 mmol) and DIEA (0.8 mL, 4.68 mmol) is stirred in 5.0 mL of DMF at room temperature overnight. 50 mL of water is added to the precipitate. The mixture is allowed to stir at room temperature for one hour. They are then filtered through a Buchner funnel, air dried and triturated with 10.0 mL of CH₃OH for 45 minutes and dried. 0.34 g of N-{3-[4-(3-Methoxyphenyl)-pyrimidin-2-ylamino]-4-methyl-phenyl}-3-trifluoromethyl-benzamide is obtained as an off-white solid.

¹H NMR (DMSO, 500MHz): δ (ppm) 10.43 (s, 1H), 8.90 (s, 1H), 8.48 (d, 1H,J=4.94Hz), 8.30( m,1H), 8.27 (d, 1H, J=8.40 Hz), 8.11 (m, 1H), 7.97 (d, 1H, J=7.39 Hz), 7.79(t, 1H,J=8.02Hz), 7.71-7.67 (br m, 2H), 7.51 (dd, 1H,J₁=8.41 Hz, J₂=2.00 Hz), 7.40 (t,1H, J=8.20 Hz), 7.36(d, 1H, J=5.21 Hz), 7.07(d, 1H,J₁= 8.40 Hz, J₂=2.40 Hz), 3.74(s,3H), 2.26 (s, 3H).

API-MS m/z 479.2 ([M+H]⁺, calcd 479.2)

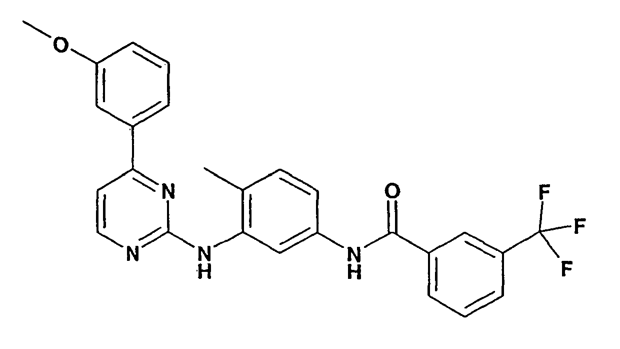

### Example 11

### 3-Diethylamino-N-[4-methyl-3-(4-pyrazin-2-yl-pyrimidin-2-ylamino)-phenyl]-benzamide

A mixture of 4-Methyl-N*3*-(4-pyrazin-2-yl-pyrimidin-2-yl)-benzene-1,3-diamine (0.55 g, 1.98 mmol), 3-Diethylamino-benzoic acid (0.48 g, 2.08 mmol), HCl, BOP (1.05 g, 2.38 mmol) and DIEA (1.7 mL, 9.50 mmol) is stirred in 6.0 mL of DMF at room temperature overnight. 50 mL of water is added to the precipitate. The resulting suspension is allowed to stir at room temperature for one hour. The reaction mixture is then filtered through a Buchner funnel, air dried and triturated with 10.0 mL of CH₂OH for 1 hour and dried. 0.87 g of 3-Diethylamino-N-[4-methyl-3-(4-pyrazin-2-yl-pyrimidin-2-ylamino)-phenyl]-benzamide is obtained as a pale yellow solid.

¹H NMR (DMSO, 500MHz): δ (ppm) 10.07 (s, 1H), 9.50 (s, 1H), 9.07 (s, 1H), 8.78 (s, 2H), 8.62 (d, 1H, J=4.78 Hz), 8.17 (m, 1H), 7.62 (d, 1H, J=5.76 Hz), 7.50(dd, 1H, J₁=8.19 Hz, J₂=2.16 Hz), 7.29-7.21 (br m, 2.H), 7.17-7.11 (br m, 2H), 6.84 (dd, 1H, J₁=7.8 Hz, J₂=2.44 Hz ), 3.39(q, 4H, J=5.99 Hz), 2.25(s,3H), 1.11 (t, 6H, J=6.99 Hz).

API-MS m/z 454.1 ([M+H]⁺, calcd 454.2)

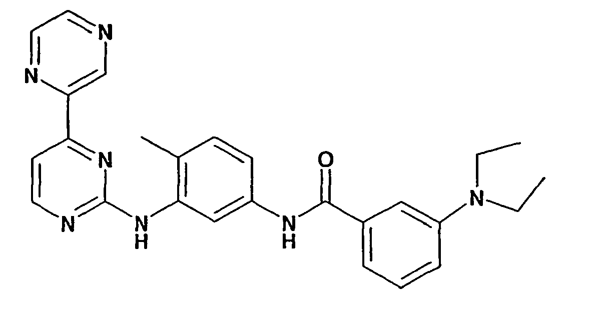

### Example 12

### N-[4-Methyl-3-(4-pyrazin-2-yl-pyrimidin-2-ylamino)-phenyl]-3-trifluoromethoxy-benzamide

A mixture of 4-Methyl-N*3*-(4-pyrazin-2-yl-pyrimidin-2-yl)-benzene-1,3-diamine (0.55 g, 1.98 mmol), 3-Trifluoromethoxy-benzoic acid (0.43 g, 2.08 mmol), BOP (1.05 g, 2.38 mmol) and DIEA (1.25 mL, 7.20 mmol) is stirred in 6.0 mL of DMF at room temperature overnight. 50 mL of water is added to the precipitate. The mixture is allowed to stir at room temperature for one hour. The mixture is then filtered through a Buchner funnel, air dried and triturated with 10.0 mL of CH₃OH for 45 minutes and dried. 0.91 g of N-[4-Methyl-3-(4-pyrazin-2-yl-pyrimidin-2-ylamino)-phenyl]-3-trifluoromethoxy-benzamide is obtained as a pale yellow solid.

¹H NMR (DMSO, 500MHz): δ (ppm) 10.37 9s, 1H), 9.49 9s, 1H), 9.10 (s, 1H), 8.79 (s, 2H), 8.62 (d, 1H, J=5.24 Hz), 8.16 (m, 1H), 8.03 (d, 1H, J=8.09), 7.93 (br s, 1H),7.69 (t, 1H, J=8.02 Hz), 7.63 (d, 1H, 5.15 Hz), 7.60 (br d, 1H, J=8.48 Hz),7.50 (dd, 1H, J₁=8.17 Hz, J₂=2.12 Hz), 7.25 (d, 1H, J=8.48 Hz), 2.26 (s, 3H).

API-MS m/z 467.2 ([M+H]⁺, calcd 467.1)

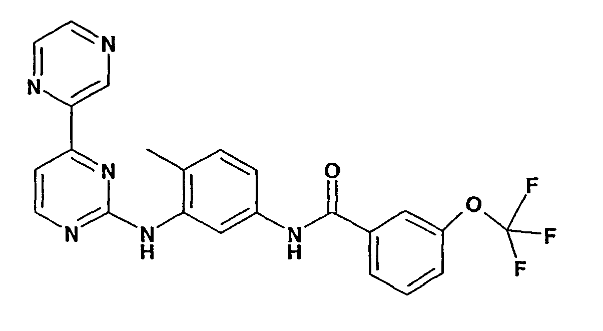

### Example 13

### N-{3-[4-(3-Ethyl-pyrazin-2-yl)-pyrimidin-2-ylamino]-4-methyl-phenyl}-3-trifluoromethyl-benzamide

A mixture of N*3*-[4-(3-Ethyl-pyrazin-2-yl)-pyrimidin-2-yl]-4-methyl-benzene-1,3-diamine (0.25 g, 0.82 mmol), 3-Trifluoromethyl-benzoic acid (0.16 g, 0.86 mmol), BOP (0.43 g, 0.98 mmol) and DIEA (0.5 mL, 2.95 mmol) is stirred in 3.0 mL of DMF at room temperature overnight. 50 mL of water is added to the precipitate. The resulting suspension is allowed to stir at room temperature for one hour. It is then filtered through a Buchner funnel, air dried and triturated with 2.0 mL of CH₃OH for 1 hour and dried. 0.18 g of N-{3-[4-(3-Ethyl-pyrazin-2-yl)-pyrimidin-2-ylamino]-4-methyl-phenyl}-3-trifluoromethyl-benzamide is obtained as an off-white solid.

¹H NMR (DMSO, 500MHz): δ (ppm) 10.40 (s, 1H), 9.05 (s, 1H), 8.64 (d, 1H, J=2.34Hz), 8.56 (m,2H), 8.28 (m, 1H), 8.25 (d, 1H, J=8.47Hz), 7.96 (d, 1H, J=8.09 Hz), 7.87(d, 1H,J=2_{.}30 Hz), 7.78 (t, 1H, J=7.36Hz), 7.53 (dd, 1H,J₁=8.82 Hz,J₂=2.30 Hz), 7.23 (m, 2H), 2.97(q,2H,J=5.8Hz), 2.22(s,3H), 1.01(t, 3H, J=8.7Hz).

API-MS m/z 479.5 ([M+H]⁺, calcd 479.5)

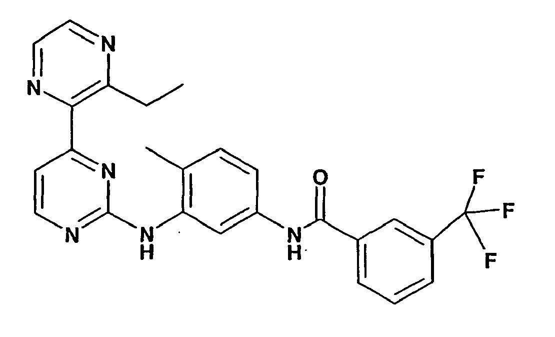

### Example 14

### N-(4-Methyl-3-{4-[6-(3-morpholin-4-yl-propylamino)-pyridin-3-yl]-pyrimidin-2-ylamino}-phenyl)-3-trifluoromethyl-benzamide

N-(3-[4-(6-Chloro-pyridin-3-yl)-pyrimidin-2-ylamino]-4-methyl-phenyl)-3-trifluoromethyl-benzamide (500mg, 1.03mmoles) is stirred in 5mL of 3-Morpholin-4-yl-propylamine at 110 °C overnight. The reaction mixture is diluted with water. The yellow precipitate is collected by filtration, triturated with dichloromethane and diethyl ether and dried under vacuum. A white solid is obtained (275mg, yield 45.2%).
¹H NMR (DMSO, 300MHz): δ (ppm)
10.43 (s, 1H), 9.40 (s, 1H), 8.76 (d, J=2.26 Hz, 1H), 8.69 (s,1H), 8.32 (d, J=5.27 Hz, 1H), 8.27 (d, J=9.80, 2H), 8.10 (d, J=2.26, 1H), 8.07 (m, 1H), 7.96 (d, J=7.9 Hz, 1H), 7.78 (t, J=7.72 Hz, 1H), 7.47(dd, J=2.02, 8.29 Hz, 1H), 7.21 (d, J=13.19 Hz, 1H), 7.20 (s, 1H), 7.12 (t, J=5.27 Hz, 1H), 6.50 (d, J=9.04 Hz, 1H), 3.57-3.54 (m, 4H), 3.35-3.25 (m, 2H), 2.40-2.25 (m, 6H), 2.23 (s, 3H), 1.68 (m, 2H)
API-MS m/z 592.3([M+H]⁺, calcd 592.3)

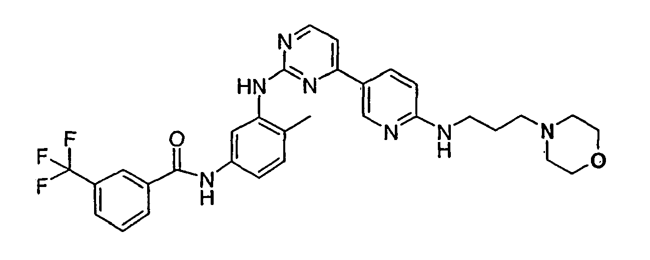

### Example 15

### N-(4-Methyl-3-(4-[6-(pyridin-4-ylamino)-pyridin-3-yl]-pyrimidin-2-ylamino}-phenyl)-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) and Pyridin-4-ylamine (0.5g, 5.3 mmoles) are dissolved in 1 mL of DMSO at room temperature, then heated to 110 °C and stirred overnight. The reaction mixture is diluted with water. The precipitate is collected by filtration, triturated with dichloromethane and methanol and dried under vacuum. A white solid is obtained (8.5mg, yield 15.2%).
¹H NMR (DMSO, 300MHz): δ (ppm)
10.49 (s, 1H), 9.33 (d, J=2.26 Hz, 1H), 9.10 (s, 1H), 8.91 (d, J=7.54 Hz,2H), 8.875-8.80 (m, 3H), 8.59 (d, J=4.90, 1H), 8.29 (d, J=7.9 Hz, 2H), 8.15 (d, J=1.88 Hz, 1H), 8.08(d, J=8.67 Hz, 1H), 7.97 (t, J=7.53 Hz, 1H), 7.79 (t, J=7.54 Hz, 1H), 7.56 (d, J=5.27 Hz, 1H), 7.49 (dd, J=8.29, 1.88 Hz, 1H), 7.25 (d, J=8.29 Hz, 1H), 7.02 (d, J=7.91 Hz, 2H), 2.23 (s, 3H).
API-MS m/z 542.4([M+H]⁺, calcd 542.5)

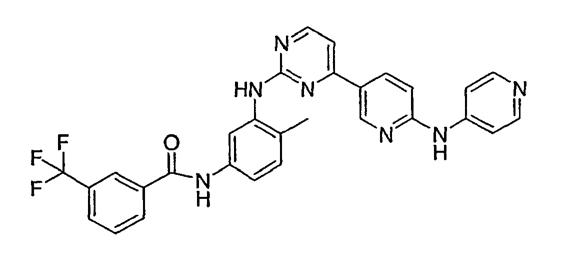

### Example 16

### 3-Difluoromethoxy-N-(3-{4-[6-(2-hydroxy-ethylamino)-pvridin-3-yl]-pyrimidin-2-ylamino}-4-methyl-phenyl)-benzamide

N-{3-[4-(6-Chloro-pyridin-3-yl)-pyrimidin-2-ylamino]-4-methyl-phenyl}-3-difluoromethoxy-benzamide (160mg, 0.331mmoles) is stirred in 2ml of 2-Amino-ethanol at 110 °C overnight. The reaction mixture is diluted with water and extracted with ethyl acetate. The combined organic phase is concentrated. The dark residue triturated with a mixture of dichloromethane and diethyl ether and methanol and dried under vacuum. A light yellow solid is obtained (66mg, yield 39.3%).
¹H NMR (DMSO, 300MHz): δ (ppm)
10.31 (s, 1H), 8.75 (d, J=2.26 Hz, 1H), 8.69 (s,1H), 8.32 (d, J=5.65 Hz, 1H), 8.09 (dd, J=9.61, 2.64 Hz, 2H), 7.86 (d, J=7.91 Hz, 1H), 7.65-7.55 (m, 1H), 7.45 (ddd, J=27.97, 8.01, 1.79 Hz, 1H), 7.38 (m, 1H), 7.23-7.12(m, 3H), 6.56 (d, J=8.67 Hz, 1H), 4.80 (t, J=4.71 Hz, 1H), 4.15 (q, J=5.27 Hz, 1H), 3.53 (q, J=5.77 Hz, 2H), 3.16(d, J=5.27 Hz, 1H), 2.21 (s, 3H)
API-MS m/z 507.2([M+H]⁺, calcd 507.2)

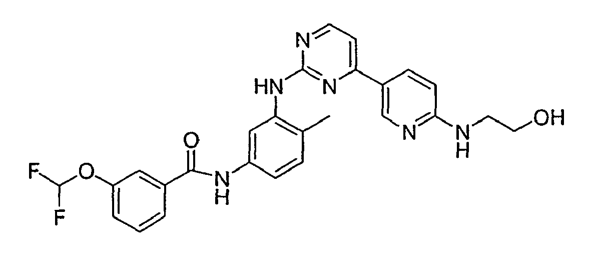

### Example 17

### N-{3-[4-(6-Chloro-pyridin-3-yl)-pyrimidin-2-ylamino]-4-methyl-phenyl}-3-trifluoromethyl-benzamide

N*3*-[4-(6-Chloro-pyridin-3-yl)-pyrimidin-2-yl]-4,N*1*-dimethyl-benzene-1,3-diamine (1.34g, 4.298 mmoles), 3-trifluoromethyl-benzoic acid (0.8575g, 4.51 mmoles), BOP (2.28g, 5.16mmoles) are dissolved in 10 mL of DMF. Then DIEA (2.8 ml, 15.47 moles) is added to above mixture and the mixture is stirred at room temperature overnight. The dark reaction mixture is diluted with water. The precipitate is washed with water, saturated sodium carbonate, water and cold methanol. The resulting product is a yellow solid (1.66g, yield 80%).
¹H NMR (DMSO, 300MHz): δ (ppm)
10.44 (s, 1H), 9.13 (d, J=2.26 Hz, 1H), 9.05 (s,1H), 8.57-8.50 (m, 2H), 8.28 (d, J=11.68 Hz, 2H), 8.10 (d, J=1.89 Hz, 1H), 7.97 (d, 7.91 Hz, 1H), 7.79 (t, J=7.91 Hz, 1H), 7.66 (d, J=8.29 Hz, 1H), 7.51-7.42 (m, 2H), 7.24 (d, J=8.29 Hz, 1H), 2.23 (s, 3H)
API-MS m/z 484.37, 486.45 ([M+H]⁺, [(M+2)+H]⁺, calcd 483.12, 486.12)

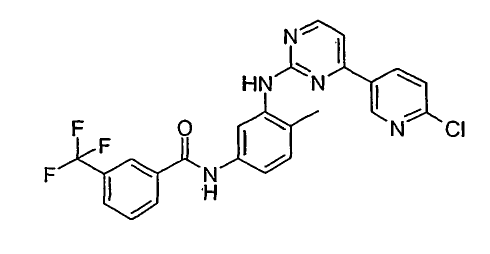

### Example 18

### N-{3-[4-(6-Dimethylamino-pyridin-3-yl)-pyrimidin-2-ylamino]-methyl-phenyl}-3-trifluoromethyl-benzamide

N*3*-[4-(6-Dimethylamino-pyridin-3-yl)-pyrimidin-2-yl]-4-methyl-benzene-1,3-diamine (0.2 g, 0.6242 mmoles), 3-trifluoromethyl-benzoic acid (0.1246g, 0.655 mmoles), BOP (0.331 g, 0.749mmoles) are dissolved in 5 mL of DMF. DIEA (0.4 mL, 2.247 mmoles) is added and the mixture is stirred at room temperature overnight. The dark reaction mixture is diluted with water. The precipitate is chromatographed on silica gel with 80 % ethyl acetate hexanes. The resulting product is a yellow solid (180mg, yield 61 %).
¹H NMR (DMSO, 300MHz): δ (ppm)
10.44 (s, 1H), 8.86 (d, J=2.26 Hz, 1H), 8.72 (s,1H), 8.34 (d, J=5.27 Hz, 1H), 8.28 (d, J=9.42 Hz, 2H), 8.21 (dd, J=9.04, 2.26 Hz, 1H), 8.09 (d, J=2.26 Hz, 1H), 7.96 (d, J=7.91 Hz, 1H), 7.78 (t, J=7.72 Hz, 1H), 7.48 (dd, J=8.10, 2.07 Hz, 1H), 7.28-7.19 (m, 2H), 6.68 (d, J=9.04 Hz, 1H), 3.09 (s, 6H), 2.23 (s, 3H)
API-MS m/z, 493.5 ([M+H]⁺, calcd 493.5)

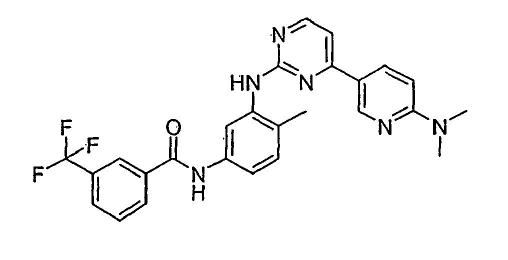

### Example 19

### N-(3-{4-[6-(2-Methoxy-ethoxy)-pyridin-3-yl]-pyrimidin-2-ylamino}-4-methyl-phenyl)-3-trifluoromethyl-benzamide

NaH (90.9mg, 2.27mmoles, 60%) is added to the solution of 2-Methoxy-ethanol (78.6mg, 1.033mmoles) at room temperature. The reaction mixture is kept stirring at room temperature for 30 min before N-{3-[4-(6-Chloro-pyridin-3-yl)-pyrimidin-2-ylamino]-4-methylphenyl}-3-trifluoromethyl-benzamide (50mg, 0.1033mmoles) is added. The reaction mixture is further stirred at room temperature for another 4 hours. The reaction mixture is diluted with water and the precipitate filtered to obtain a white solid (64mg, 100%).
¹H NMR (DMSO, 300MHz): δ(ppm)
10.44 (s, 1H), 8.95-8.85 (m, 2H), 8.55-8.35 (m,2H), 8.30-8.24 (m, 2H), 8.10 (s, 1H), 7.97 (d, J=7.92 Hz, 1H), 7.79 (t, J=7.54 Hz, 1H), 7.45 (d, J=8.29Hz, 1H), 7.36 (d, J=4.90 Hz, 1H), 7.23 (d, J=8.29 Hz, 1H), 6.93 (d, J=8.67 Hz, 1H). 4.43 (t, J=4.52 Hz, 2H), 3.66 (t, J=4.52 Hz, 2H), 3.29 (s, 3H), 2.23 (s, 3H)
API-MS m/z 524.5 ([M+H]⁺, calcd 524.5

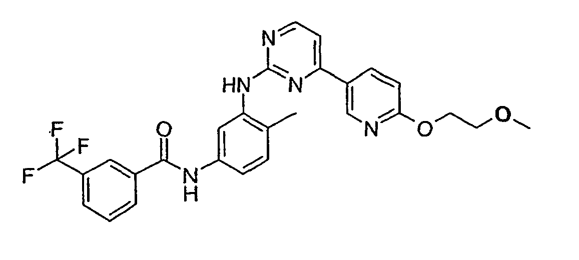

### Example 20

### N-(4-Methyl-3-{4-[6-(4-methyl-piperazin-1-yl)-pyridin-3-yl]-pyrimidin-2-ylamino}-phenyl)-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1g of 1-Methyl-piperazine at 110°C overnight. The reaction mixture is diluted with water and the yellow precipitate is collected by filtration. After drying under vacuum, a white solid is obtained (55mg, yield 97.2%).
¹H NMR (DMSO, 300MHz): δ(ppm)
10.44 (s, 1H), 8.95-8.85 (m, 2H), 8.55-8.35 (m,2H), 8.30-8.24 (m, 2H), 8.10 (s, 1H), 7.97 (d, J=7.92 Hz, 1H), 7.79 (t, J=7.54 Hz, 1H), 7.45 (d, J=8.29Hz, 1H), 7.36 (d, J=4.90 Hz, 1H), 7.23 (d, J=8.29 Hz, 1H), 6.93 (d, J=8.67 Hz, 1H), 4.43 (t, J=4.52 Hz, 2H), 3.66 (t, J=4.52 Hz, 2H), 3.29 (s, 3H), 2.23 (s, 3H)
API-MS m/z 524.5 ([M+H]⁺, calcd 524.5

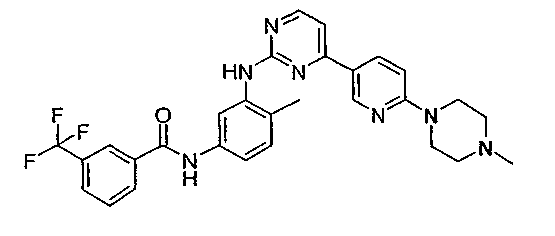

### Example 21

### N-{4-Methyl-3-[4-(6-methylamino-pyridin-3-yl)-pyrimidin-2-ylamino]-phenyl}-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 2 mL of methyl amine (2 M in THF) at 110 °C overnight. The reaction mixture is diluted with water and the yellow precipitate is collected by filtration. After drying under vacuum, a white solid is obtained (31 mg, yield 63%).
¹H NMR (DMSO, 300MHz): δ (ppm)
10.44 (s, 1H), 8.78( d, J=2.26Hz, 1H), 8.70( s, 1H), 8.35-8.24 ( m, 3H), 8.15-8.05 (m, 2H), 7.97( d, J=1.87 Hz, 1H), 7.78 (t, J = 7.72 Hz, 1H), 7.47 (dd, J= 8.10, 2.07 Hz, 1H), 7.24-7.18 (m, 2H), 7.10-7.00 (m, 1H), 6.49 (d, J=8.67Hz, 1H), 2.81 (d, J=4.52 Hz, 3H), 2.23 (s, 3H)
API-MS m/z 479.2 ([M+H]⁺, calcd 479.2

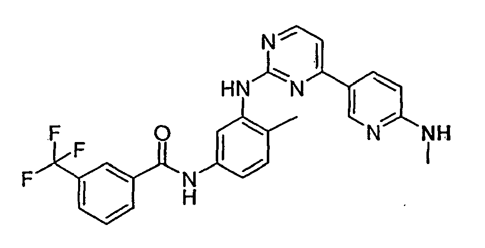

### Example 22

### N-{3-[4-(6-Cyclopropylamino-pyridin-3-yl)-pyrimidin-2-ylaminol-4-methyl-pheny}-3-trifluoromethyl-benzamide

N-{3-[4-(6-Chloro-pyridin-3-yl)-pyrimidin-2-ylamino]-4-methyl-pheny}-3-trifluoromethyl-benzamide (50mg, 0.103mmoles) is stirred in 1mL of Cyclopropylamine at 110 °C overnight. The reaction mixture is diluted with water. The precipitate is collected by filtration and recrystallized in ether. After drying under vacuum, a white solid is obtained (6mg, yield 11.5%).
¹H NMR (DMSO, 300MHz): δ (ppm)
10.44 (s, 1H), 8.78( d, J=2.26Hz, 1H), 8.70( s, 1H), 8.35-8.24 ( m, 3H), 8.19 (dd, J = 8.85, 2.45 Hz, 1H), 8.08( d, J=1.88 Hz, 1H), 7.96 (d, J=7.91 Hz, 1H), 7.78 (t, J = 7.72 Hz, 1H), 7.47 (dd, J= 8.10, 1.70 Hz, 1H), 7.33 (d, J=2.26 Hz, 1H), 7.50-7. 40 (m, 2H), 6.63 (d, J=8.67Hz, 1H), 2.60-2.53 (m, 1H), 2.23 (s, 3H), 0.76-0.68 (m, 2H), 0.48-0.40 (m, 2H)
API-MS m/z 505.3 ([M+H]⁺, calcd 505.2

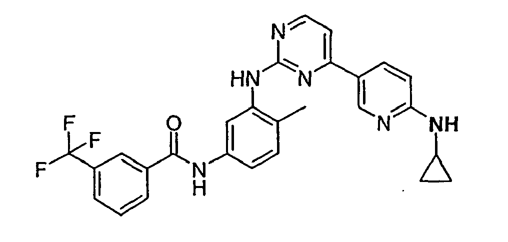

### Example 23

### N-{3-[4-(6-Cyclopentylamino-pyridin-3-yl)-pyrimidin-2-ylamino]-4-methyl-phenyl}-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1ml of Cyclopentylamine at 110 C° overnight. The reaction mixture is diluted with water. The precipitate is collected by filtration and recrystallized from ether. After drying under vacuum, a white solid is obtained (27mg, yield 49.2%).
¹H NMR (DMSO, 300MHz): δ (ppm)
10.44 (s, 1H), 8.76( d, J=2.26Hz, 1H), 8.67( s, 1H), 8.36-8.24 ( m, 3H), 8.13-8.04 (m, 2H), 7.97 ( d, J=7.91 Hz, 1H), 7.78 (t, J = 7.72 Hz, 1H), 7.47 (dd, J= 8.28, 1.88 Hz, 1H), 7.25-7.15 (m, 2H),7.11-7.04 (m, 1H), 6.48 (d, J=9.04 Hz, 1H), 4.21-4.05 (m, 1H), 2.23 (s, 3H), 2.00-1.85(m, 2H), 1.75-1.35 (m 6H)
API-MS m/z 533.3 ([M+H]⁺, calcd 533.2

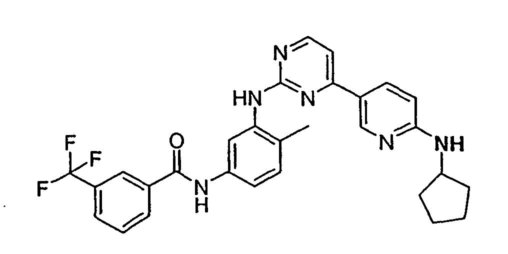

### Example 24

### N-{4-Methyl-3-[4-(6-thiomorpholin-4-yl-pyridin-3-yl)-pyrimidin-2-ylaminol-phenyl}-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1mL of Thiomorpholine at 110 °C overnight. The reaction mixture is diluted with water and the precipitate is collected by filtration. After drying under vacuum, a white solid is obtained (50mg, yield 87.9%).
¹H NMR (DMSO, 300MHz): δ (ppm)
10.44 (s, 1H), 8.87 (d, J=2.26Hz, 1H), 8.76 ( s, 1H), 8.36 ( d, J = 5.27 Hz, 1H), 8.32-8.20 (m, 3H), 8.07 ( d, J=1.88 Hz, 1H), 7.96 (d, J =7.16 Hz, 1H), 7.78 (t, J = 7.91 Hz, 1H), 7.47 (dd, J= 8.10, 2.07 Hz, 1H), 7.28-7.18 (m, 2H),7.11-7.04 (m, 1H), 6.91 (d, J=9.04 Hz, 1H), 4.25-3.94 (m, 4H), 2.65-2.56(m, 4H), 2.23 (s, 3H)
API-MS m/z 551.5 ([M+H]⁺, calcd 551.3

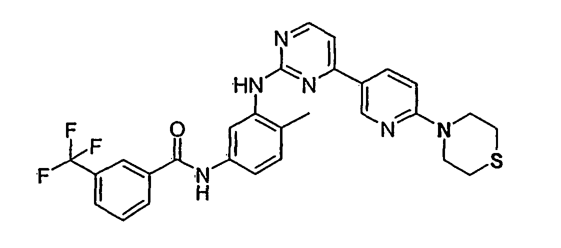

### Example 25

### N-{4-Methyl-3-[4-(6-morpholin-4-yl-pyridin-3-yl)-pyrimidin-2-ylaminol-phenyl}-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1mL of morpholine at 110 °C overnight. The reaction mixture is diluted with water and the precipitate is collected by filtration. After drying under vacuum, a white solid is obtained (46mg, yield 83.3%).
¹H NMR (DMSO, 300MHz): δ (ppm)
10.44 (s, 1H), 8.89 ( d, J=1.88Hz, 1H), 8.77 ( s, 1H), 8.36 ( d, J = 5.27 Hz, 1H), 8.32-8.20 (m, 3H), 8.08 (d, J=1.88 Hz, 1H), 7.96 (d, J =7.53 Hz, 1H), 7.78 (t, J = 7.72 Hz, 1H), 7.48 (dd, J= 8.29, 2.26 Hz, 1H), 7.28-7.18 (m, 2H), 6.90 (d, J=8.67 Hz, 1H), 3.75-3.65 (m, 4H), 3.60-3.5(m, 4H), 2.23 (s, 3H)
API-MS m/z 535.5 ([M+H]⁺, calcd 535.3

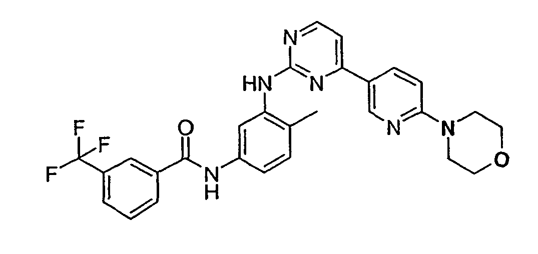

### Example 26

### N-{3-[4-(4-Hydroxy-3,4,5,6-tetrahydro-2H-[1,2']bipyridinyl-5'-yl)-pyrimidin-2-ylamino]-4-methyl-phenyl}-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1mL of Piperidin-4-ol at 110 °C overnight. The reaction mixture is diluted with water and the precipitate is collected by filtration. After drying under vacuum, a white solid is obtained (54 mg, yield 98.4%).
¹H NMR (DMSO, 300MHz): δ (ppm)
10.44 (s, 1H), 8.86 (d, J=1.88Hz, 1H). 8.73 (s,1H), 8.43-8.18 (m, 4H), 8.10 (s, 1H),7.96(d, J =7.91 Hz, 1H), 7.78 (t, J = 7.53 Hz, 1H), 7.48 (dd, J= 8.47, 0.94 Hz, 1H), 7.28-7.18 (m, 2H), 6.90 (d, J=9.04 Hz, 1H), 4.75-4.69 (m, 1H), 4.15-4.00(m, 2H), 3.79-3.66 (m, 1H), 3.29-3.15 (m, 2H),2.23 (s, 3H), 1.84-1.71 (m, 2H), 1.42-1.20 (m, 2H);
API-MS m/z 549.5 ([M+H]⁺, calcd 549.3

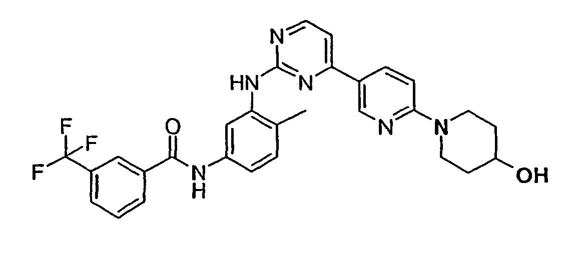

### Example 27

### N-(3-{4-[6-(3-Diethylamino-propylamino)-pyridin-3-yl]-pyrimidin-2-ylamino}-4-methyl-phenyl)-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1ml of N*1*,N*1*-Diethyl-propane-1,3-diamine at 110°C overnight. The reaction mixture is diluted with water and the precipitate is collected by filtration. After drying under vacuum, a white solid is obtained (80 mg, yield 100%).
¹H NMR (DMSO, 300MHz): δ (ppm)
10.50 (s, 1H), 8.76 (d, J=1.88Hz, 1H), 8.69 (s, 1H), 8.32-8.25 (m, 3H), 8.13-8.05 (m, 2H), 7.96 (d, J =7.53 Hz, 1H), 7.78 (t, J = 8.10 Hz, 1H), 7.48 (dd, J= 8.10, 1.70 Hz, 1H), 7.25-7.18 (m, 2H), 6.50 (d, J=9.04 Hz, 1H), 3.33-3.23 (m, 2H), 2.5-2.35(m, 6H), 2.23 (s, 3H), 1.03-0.85(m, 6H)
API-MS m/z 578.6 ([M+H]⁺ calcd 578.4

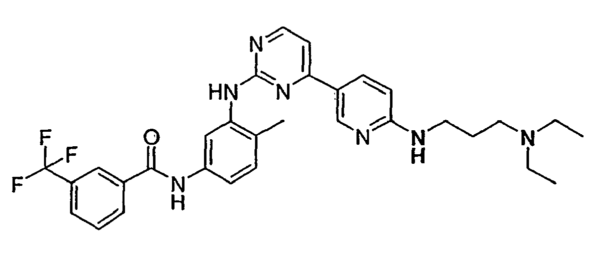

### Example 28

### N-[4-Methyl-3-(4-{6-[(pyridin-4-ylmethyl)-amino]-pyridin-3-yl}-pyrimidin-2-ylamino)-phenyl]-3--trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1 mL of C-Pyridin-4-yl-methylamine at 110 C° overnight. The reaction mixture is diluted with water. The precipitate is collected by filtration and washed with ether. After drying under vacuum, a white solid is obtained (30 mg, yield 52.4%).
¹H NMR (DMSO, 300MHz): δ (ppm)
10.42 (s, 1H), 8.73 (d, J=1.88Hz, 1H), 8.70 (s, 1H), 8.46 (d, J = 6.03 Hz, 2H), 8.32 (d, J=5.27 Hz, 1H), 8.31-8.23 (m, 2H), 8.13 (dd, J=8.67, 2.26 Hz, 1H), 8.05(d, J =1.88 Hz, 1H), 7.96 (d, J = 7.91 Hz, 1H), 7.82-7.69 (m, 2H), 7.46 (dd, J= 8.29, 2.26 Hz, 1H), 7.28 (d, J=5.65, 2H), 7.24-7.15 (m, 2H), 6.61 (d, J=8.67 Hz, 1H), 4.57 (d, J=5.65 Hz, 2H), 2.23 (s, 3H)
API-MS m/z 556.5 ([M+H]⁺, calcd 556:5

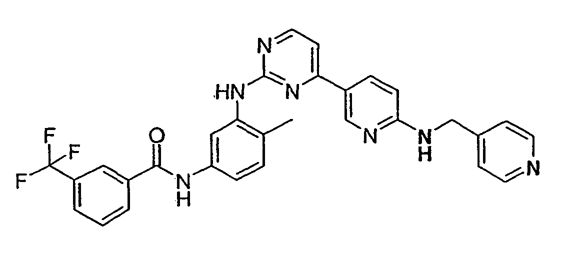

### Example 29

### N-(3-{4-[6-(2-Methoxy-ethylamino)-pyridin)-3-yl]-pyrimidin-2-ylamino}-4-methyl-phenyl)-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.1 03mmoles) is stirred in 1 mL of 2-Methoxy-ethylamine at 110 °C overnight. The reaction mixture is diluted with water and the precipitate is collected by filtration and washed with ether. After drying under vacuum, a yellow solid is obtained (65 mg, yield 66.9%).
¹H NMR (DMSO, 300MHz): δ (ppm)
10.42 (s, 1H), 8.76 (d, J=2.26 Hz, 1H), 8.69 (s, 1H), 8.35-8.25 (m, 3H), 8.13-8.05 (m, 2H), 7.96 (d, J =7.96 Hz, 1H), 7.78 (t, J = 7.72 Hz, 1H), 7.48 (dd, J= 8.10, 2.07 Hz, 1H), 7.25-7.13 (m, 3H), 6.56 (d, J=9.04 Hz, 1H), 3.48-3.43 (m, 4H), 3.26(s, 3H),3.17 (d, J=5.27 Hz, 2H),2.23 (s, 3H)
API-MS m/z 523.5 ([M+H]⁺, calcd 523.5

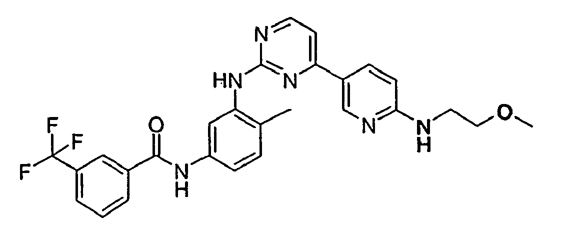

### Example 30

### N-(3-{4-r6-(2-Hydroxy-propylsulfanyl)-pyridin-3-yl]-pyrimidin-2-ylamino}-4-methyl-phenyl)-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles) is stirred in 1mL of 1-Mercapto-propan-2-of in the presence of potassium carbonate at room temperature overnight. The reaction mixture is diluted with water. The precipitate is collected by filtration and washed with ether. After drying under vacuum, a solid was obtained (11 mg, yield 20%).
1H NMR (DMSO, 300MHz): δ (ppm)
10.44 (s, 1H), 9.12 (d, J=2.26 Hz, 1H), 8.94 (s, 1H), 8.47 (d, J=5.27 Hz, 1H), 8.35-8.25 (m, 3H), 8.08 (d, J=1.88 Hz, 1H), 7.96 (d, J =7.91 Hz, 1H), 7.78 (t, J = 7.72 Hz, 1H), 7.48 (dd, J= 8.28, 1.88 Hz, 1H), 7.45-7.35 (m, 2H), 7.23 (d, J=8.28 Hz, 1H), 4.99 (br, 1H), 3.90-3.80(m, 1H),3.22 (d, J=6.02 Hz, 2H),2.23 (s, 3H), 1.14 (d, J=6.02 Hz, 3H)
API-MS m/z 540.4 ([M+H]⁺, calcd 540.5

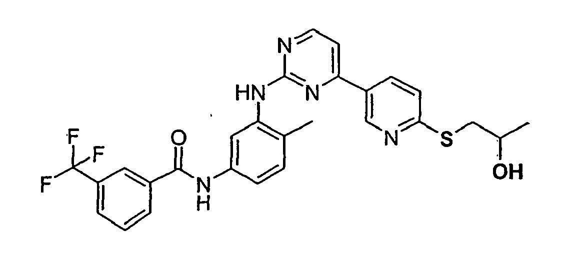

### Example 31

### N-(4-Methyl-3-{4-[6-(pyridin-3-yloxy)-pyridin-3yl]-pyrimidin-2-ylamino}-phenyl)-3-trifluoromethyl-benzamide

N-{3-[4-(6-Chloro-pyridin-3-yl)-pyrimidin-2-ylamino]-4-methyl-phenyl}-3-trifluoromethyl-benzamide (50mg, 0.103mmoles), Pyridin-3-ol (98.7mg, 1.03mmoles), 18-crown-6 (20mg), KOH (56mg) is stirred in 1 mL of toluene at 110 °C overnight. The reaction mixture is diluted with water. The precipitate is collected by filtration and further purified by prep-TLC (100% ethyl acetate). After drying under vacuum, a white solid is obtained (11.3 mg, yield 26.2%). ¹H NMR (DMSO, 300MHz): δ(ppm)
10.44 (s, 1H), 8.92(s, 1H), 8.86 (d, J=2.26 Hz, 1H), 8.59 (dd, J=8.67, 2.64 Hz, 1H), 8.53-8.45 (m, 3H), 8.30-8.22(m, 2H), 8.08 (d, J=1.88 Hz, 1H), 7.96 (d, J =7.91 Hz, 1H), 7.78 (t, J = 7.72 Hz, 1H), 7.69 (dq, J= 8.38, 1.38 Hz, 1H), 7.53-7.44 (m, 2H); 7.37 (d, J=5.27 Hz, 1H), 7.27-7.20 (m, 2H), 2.23 (s, 3H)
API-MS m/z 543.3 ([M+H]⁺ calcd 543.3

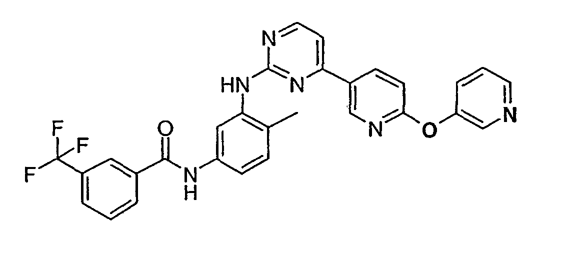

### Example 32

### N-(4-Methyl-3-{4-[6-(1-methyl-piperidin-4-yloxy)-pyridin-3-yl]-pyrimidin-2-ylamino}-phenyl)-3-trifluoromethyl-benzamide

NaH( 90.9mg, 2.27mmoles, 60%) is added to the solution of 1-Methyl-piperidin-4-ol (119mg, 1.033mmoles) in 2mL of DMF at room temperature. The reaction mixture is stirred at room temperature for 30 min before N-{3-[4-(6-Chloro-pyridin-3-yl)-pyrimidin-2-ylamino]-4-methylphenyl}-3-trifluoromethyl-benzamide (50mg, 0.1033mmoles) is added. The reaction mixture is further stirred at room temperature overnight. The reaction mixture is diluted with water and the precipitate is obtained by filtration and further purified by PREP-TLC (60% Ethyl acetate / hexanes). After drying under vacuum, a solid product is obtained (50 mg, yield 86.3%).
¹H NMR (DMSO, 300MHz): δ (ppm)
10.44 (s, 1H), 8.90 (d, J=2.26 Hz, 1H), 8.88(s, 1H), 8.46 (d, J=5.27 Hz, 1H), 8.39 (dd, J=8.85, 2.45 Hz, 1H), 8.48-8.24 (m, 2H), 8.11 (d, J=1.88 Hz, 1H), 7.96 (d, J =7.91 Hz, 1H), 7.78 (t, J = 7.72 Hz, 1H), 7.48 (dd, J= 8.28, 1.88 Hz, 1H), 7.34 (d, J=5.27 Hz, 1H), 7.23 (d, J= 8.29 Hz, 1H), 6.87 (d, J=8.67 Hz, 1H), 5.1-5.0 (m, 1H), 2.71-2.59 (m, 2H), 2.23 (s, 3H), 2.21 (s, 3H), 2.3-2.1 (m, 2H), 2.02-1.91 (m, 2H), 1.75-1.59 (m, 2H)
API-MS m/z 563.4 ([M+H]⁺, calcd 563.5

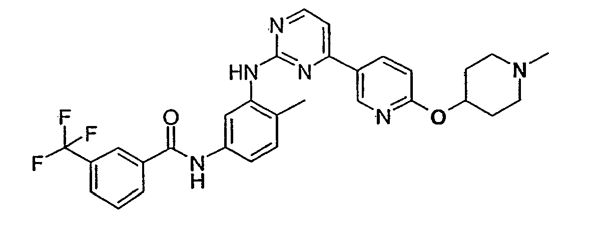

### Example 33

### N-[3-([4,5']Bipyrimidinyl)-2-ylamino)-4-methyl-phenyl]-3-trifluoromethyl-benzamide

A mixture of N*3*-[4,5']Bipyrimidinyl-2-yl-4-methyl-benzene-1,3-diamine (0.23 g, 0.83 mmol), 3-Trifluoromethyl-benzoic acid (0.16 g, 0.87 mmol), BOP (0.44 g, 1.00 mmol) and DIEA (0.5 mL, 3.00 mmol) is stirred in 3.0 mL of DMF at room temperature overnight. 40 mL of water is added to the precipitate. The resulting suspension is allowed to stir at room temperature for 1 hour. It is then filtered through a Buchner funnel, triturated twice with 5.0 mL of CH₃OH and dried. A brown solid is obtained.
The solution is purified by flash chromatography using 1:5 EtOAc:Hexane → 1:1 1 EtOAc:Hexane. The solvent is removed under vacuum. 0.14 g of N-[3-([4,5']Bipyrimidinyl-2-ylamino)-4-methyl-phenyl]-3-trifluoromethyl-benzamide is obtained as a yellow solid.
¹H NMR (DMSO, 500MHz): δ(ppm) 10.50 (s, 1H), 9.40 (s, 1H), 9.18 (s, 1H), 9.05 ( d,1H, J=3.78 Hz), 8.71 (d, 1H, J=4.16 Hz), 8.46-8.27 (m, 3H), 8.17 (s, 1H), 8.00 (d, 1H, J=7.14Hz), 7.83 (dd, 1H, J=6.7 Hz), 7.75(d, 1H, J=3.96 Hz), 7.64-7.43 (m, 2H), 7.36-7.20 (m, 1H), 2.29 (s, 3H).
API-MS m/z 451.2 ([M+H]⁺ calcd 451.1)

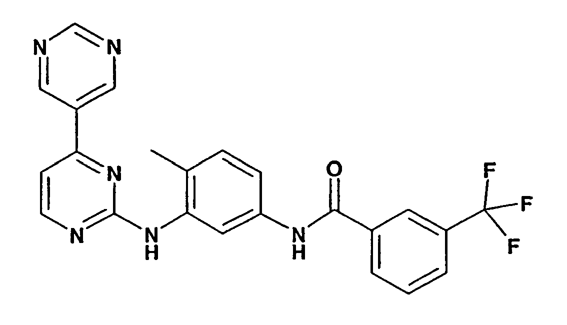

### Example 34

### 3-Fluoro-N-[4-methyl-3-(4-pyrazin-2-yl)-pyrimidin-2-ylamino)-phenyl]-5-trifluoromethyl-benzamide

A mixture of 4-Methyl-N*3*-(4-pyrazin-2-yl-pyrimidin-2-yl)-benzene-1,3-diamine (0.55 g, 1.98 mmol), 3-Fluoro-5-trifluoromethyl-benzoic acid (0.43 g, 2.08 mmol), BOP (1.05 g, 2.38 mmol) and DIEA (1.25 mL, 7.20 mmol) is stirred in 9.0 mL of DMF at room temperature overnight. 25 mL of water is added to the precipitate. The resulting suspension is allowed to stir at room temperature for one hour. It is then filtered through a Buchner funnel, air dried and triturated with 10.0 mL of CH₃OH for 1 hour and dried. 0.80 g of 3-Fluoro-N-[4-methyl-3-(4-pyrazin-2-yl-pyrimidin-2-ylamino)-phenyl]-5-trifluoromethyl-benzamide is obtained as a pale yellow solid.
¹H NMR (DMSO, 500MHz): δ (ppm) 10.49 (s, 1H), 9.49 (s, 1H), 9.10 (s, 1H), 8.79 ( s,2H ), 8.62 (d, 2H, J=4.81 Hz), 8.10-8.20 (m, 3H), 7.96 (d, 1H, J=8.66Hz), 7.63 (d, 1H,J=4.78Hz), 7.48 (dd, 1H,J₁=8.46Hz, J₂=2.05 Hz), 7.26 (d, 1H,J=8.09Hz), 2.26 .(s, 3H).
API-MS m/z 469.1 ([M+H]⁺, calcd 469.1)

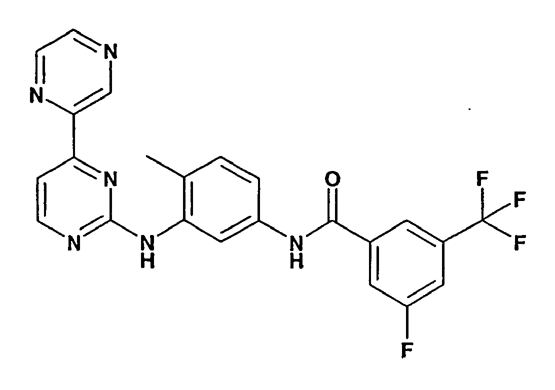

### Biological Activity

Active B-Raf, C-Raf, and V599E B-Raf proteins of human sequence are purified from insect cells using the baculoviral expression system. Raf inhibition is tested in 96-well microplates coated with IκB-α and blocked with Superblock. The phosphorylation of IκB-α at Serine 36 is detected using a phospho-IκB-α specific antibody (Cell Signaling #9246), an anti-mouse IgG alkaline phosphatase conjugated secondary antibody (Pierce # 31320), and an alkaline phosphatase substrate, ATTOPHOS (Promega, #S101 ).

Each of the compounds of Examples 1-34 inhibits wild-type C-RAF at an IC₅₀ of from 0.01 to 0.7 micromoles/liter and/or wild-type B-RAF at an IC₅₀ of from 0.04 to 1.5 micromoles/liter and/or mutant B-RAF (V599E) at an IC₅₀ of from 0.006 to 1.6 micromoles/liter.

### Example D1

Detection of T1796A Mutation in the Human B-Raf Gene
Detection Primer: GATTTTGGTCTAGCTACAGA
Second Primer: GACTTTCTAGTAACTCAGCAG

Genomic DNA is isolated from human cells from a melanoma cell line using a GENELUTE mammalian genomic DNA kit (Sigma cat # G1 N 350). PCR reactions are carried out on a PCR machine (MJ Research, Model PTC100) in a total volume of 50 microL using the PCR Core kit by Roche (cat # 1578 553). The PCR reaction mixture contains 5 microL of 10X reaction buffer,1 microL of 10 mM dNTPs, 100-1000ng of template DNA, 0.5 microL Taq polymerase (2.5-5 units), 1 microL of a 31 uM stock of each primer.

The PCR conditions are as follows:

| | | | |
|---|---|---|---|
| 95°C 3minutes | | | |
| | 94°C 1 minutes | | |
| | 50°C 30 second | 35 | |
| | | Cycles | |
| | 72°C 1 minutes | | |
| 72°C 10minutes | | | |
| 4°C | | | |

After amplification, 8 microLs of the PCR reaction mixture is mixed with 2 microLs of nucleic acid sample loading buffer [BioRad cat #161-0767]. The 10 microL sample is loaded onto a 1.5% agarose [GIBCO-BRL cat # 15510-027] gel that contains 0.3 ug/ml of ethidium bromide [Pierce cat #17898]. Molecular weight standards [100 bp DNA ladder from Invitrogen cat # 10380-012] are loaded in an adjacent lane. The DNA is separated by electrophoresis in TAE buffer (0.04 M Tris-acetate, 0.01 M EDTA. 0.02M glacial acetic acid pH8.4)[Roche cat #1666690]. Electrophoresis conditions are 120 volts for 30-40 minutes. After separation, the gel is exposed to UV light and a picture taken on an Alphalmager2000 documentation system.

Generally, two bands are detected in the gel. The faster migrating band runs ahead of the 100 bp marker and represents the primers. The DNA that results from the T1796A mutant specific PCR amplification has a predicted size of 152 bp and migrates between the 100 bp standard and the 200bp standard as predicted. The PCR amplification product is confirmed by sequencing. The presence of the PCR amplification product demonstrates that the T1796A mutation is present in the template DNA. The absence of the PCR amplification product is evidence that the mutation is absent in the tissue sample.

Other B-RAF mutations are detected by this method utilizing the detection primer and second primer indicated for the mutation in the following tables:

| SEQ ID NO: | Detection primer oligonucleotide segment (5'→3') | B-RAF mutation |
|---|---|---|
| 1 | ACAGTGGGACAAAGAATTGA | G1388A |
| 2 | ACAGTGGGACAAAGAATTGT | G1388T |
| 3 | GGACAAAGAATTGGATCTGC | G1394C |
| 4 | GGACAAAGAATTGGATCTGA | G1394A |
| 5 | GGACAAAGAATTGGATCTGT | G1394T |
| 6 | ATTGGATCTGGATCATTTGC | G1403C |
| 7 | ATTGGATCTGGATCATTTGA | G1403A |
| 8 | GAGTAATAATATATTTCTTCATA | G1753A |
| 9 | CAGTAAAAATAGGTGATTTG | T1782G |
| 10 | CAGTAAAAATAGGTGATTTTC | G1783C |
| 11 | GTAAAAATAGGTGATTTTGGTG | C1786G |
| 12 | GTAAAAATAGGTGATTTTGGTCG | T1787G |
| 13 | GATTTTGGTCTAGCTACAGA | T1796A |
| 14 | GATTTTGGTCTAGCTACAGAT | TG1796-97AT |

| SEQ ID NO: | Second Primer oligonucleotide segment (5'→3') | B-RAF mutation |
|---|---|---|
| 15 | TGTCACCACATTACATACTTACC | G1388A |
| 16 | TGTCACCACATTACATACTTACC | G1388T |
| 17 | TGTCACCACATTACATACTTACC | G1394C |
| 18 | TGTCACCACATTACATACTTACC | G1394A |
| 19 | TGTCACCACATTACATACTTACC | G1394T |
| 20 | TGTCACCACATTACATACTTACC | G1403C |
| 21 | TGTCACCACATTACATACTTACC | G1403A |
| 22 | GACTTTCTAGTAACTCAGCAG | G1753A |
| 23 | GACTTTCTAGTAACTCAGCAG | T1782G |
| 24 | GACTTTCTAGTAACTCAGCAG | G1783C |
| 25 | GACTTTCTAGTAACTCAGCAG | C1786G |
| 26 | GACTTTCTAGTAACTCAGCAG | T1787G |
| 27 | GACTTTCTAGTAACTCAGCAG | T1796A |
| 28 | GACTTTCTAGTAACTCAGCAG | TG1796-97AT |

## Claims

1. A compound of the formula (I) 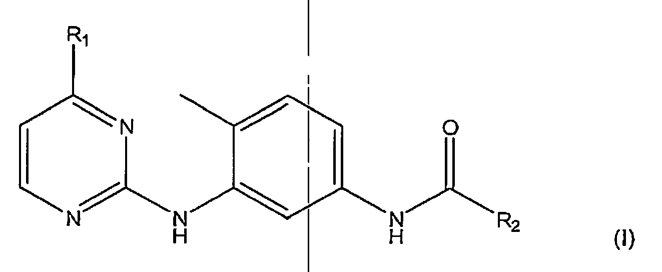 wherein
R₁ is a phenyl radical that is unsubstituted or substituted by one substituent or a heteroaryl radical selected from a thiazolyl radical, a pyrazinyl radical and a pyrimidinyl radical, each of which is unsubstituted or substituted by one substituent, or is a 6-substituted 3-pyridyl; and R₂ is phenyl that is substituted in the 3-position by fluorine, halo-lower alkyl, halo-lower alkoxy or halo-lower alkylthio;
where the term "lower" refers to a radical having up to and including a maximum of 7 carbon atoms;
or an N-oxide or a pharmaceutically acceptable salt thereof.

2. A compound of claim 1 wherein R₁ is a phenyl, 2-thiazolyl, 2-pyrazinyl, 5-pyrimidinyl or 3-pyridyl radical.

3. A compound of the formula according to claim 1, selected from the group consisting of
N-[4-Methyl-3-(4-thiazol-2-yl-pyrimidin-2-ylamino)-phenyl]-3-trifluoromethyl-benzamide;
N-[4-Methyl-3-(4-pyrazin-2-yl-pyrimidin-2-ylamino)-pheny]-3-triftuoromethy-benzamide;
N-[4-Methyl-3-(4-pyrazin-2-yl-pyrimidin-2-ylamino)-phenyl]-3-(1,1,2,2-tetrafluoro-ethoxy)-benzamide;
3-Difluoromethoxy-N-[4-methyl-3-(4-pyrazin-2-yl-pyrimidin-2-ylamino)-pheny]-benzamide;
N-[4-Methyl-3-(4-pyrazin-2-yl-pyrimidin-2-ylamino)-phenyl]-3-(2,2,2-trifluoro-ethoxy)-benzamide;
N-[4-Methyl-3-(4-pyrazin-2-yl-pyrimidin-2-ylamino)-phenyl]-3-trifluoromethylsulfanylbenzamide;
N-{3-[4-(4-Methoxy-phenyl)-pyrimidin-2-ylamino]-4-methyl-phenyl}-3-trifluoromethyl-benzamide;
N-[4-Methyl-3-(4-phenyl-pyrimidin-2-ylamino)-phenyl]-3-trifluoromethyl-benzamide;
N-{3-[4-(3-Methoxy-phenyl)-pyrimidin-2-ylamino]-4-methyl-phenyl}-3-trifluoromethy-benzamide;
N-[4-Methyl-3-(4-pyrazin-2-yl-pyrimidin-2-ylamino)-phenyl]-3-trifluoromethoxy-benzamid;
N-{3-[4-(3-Ethyl-pyrazin-2-yl)-pyrimidin-2-ylamino]-4-methyl-phenyl}-3-trifluoromethyl-benzamide;
N-(4-Methyl-3-{4-[6-(3-morpholin-4-yl-propylamino)-pyridin-3-yl]-pyrimidin-2-ylamino}-phenyl)-3-trifluoromethyl-benzamide;
N-(4-Methyl-3-{4-[6-(pyridin-4-ylamino)-pyridin-3-yl]-pyrimidin-2-ylamino}-phenyl)-3-trifluoromethyl-benzamide;
3-Difluoromethoxy-N-(3-{4-[6-(2-hydroxy-ethylamino)-pyridin-3-yl]-pyrimidin-2-ylamino}-4-ethyl-phenyl)-benzamide;
N-{3-[4-(6-Chloro-pyridin-3-yl)-pyrimidin-2-ylamino]-4-methyl-phenyl}-3-trifluoromethyl-benzamide;
N-{3-[4-(6-Dimethylamino-pyridin-3-yl)-pyrmidin-2-ylamino]-4-methyl-phenyl}-3-trifluoromethyl-benzamide;
N-(3-{4-[6-(2-Methoxy-ethoxy)-pyridin-3-yl]-pyrimidin-2-ylamino}-4-methyl-pheny)-3-trifluoromethyl-benzamide;
N-(4-Methyl-3-{4-[6-(4-methyl-piperazin-1-yl)-pyridin-3-yl]-pyrimidin-2-ylamino}-phenyl)-3-trifluoromethyl-benzamide;
N-{4-Methyl-3-[4-(6-methylamino-pyridin-3-yl)-pyrimidin-2-ylamino]-phenyl}-3-trifluoromethyl-benzamide;
N-{3-[4-(6-Cyclopropylamino-pyridin-3-yl)-pyrimidin-2-ylamino]-4-methyl-phenyl}-3-trifluoromethyl-benzamide;
N-{3-[4-(6-Cyclopentylamino-pyridin-3-yl)-pyrimidin-2-ylamino]-4-methyl-phenyl}-3-trifluoromethyl-benzamide;
N-{4-Methyl-3-[4-(6-thiomorpholin-4-yl-pyridin-3-yl)-pyrimidin-2-ylamino]-pheny}-3-trifluoromethyl-benzamide;
N-{4-Methy-3-[4-(6-morpholin-4-yl-pyridin-3-yl)-pyrimidin-2-ylamino]-phenyl}-3-trifluaromethyl-benzamide;
N-{3-[4-(4-Hydroxy-3,4,5,6-tetrahydro-2H-[1,2']bipyridinyl-5'yl)-pyrimidin-2-ylamino]-4-methyl-phenyl}-3-trifluoromethyl-benzamide
N-(3-{4-[6-(3-Diethylamino-propylamino)-pyridin-3-yl]-pyrimidin-2-ylamino}-4-methyl-phenyl)-3-trifluoromethyl-benzemide;
N-[4-Methyl-3-(4-{6-[(pyridin-4-ylmethyl-amino]-pyridin-3-yl}-pyrimidin-2-ylamino)-phenyl]-3-trifluoromethyl-benzamide;
N-(3-{4-[6-(2-Methoxy-ethylamino)-pyridin-3-yl]-pyrimidin-2-ylamino}-4-methyl-phenyl)-3-trifluoromethyl-benzamide;
N-(3-{4-[6-(2-Hydroxy-propylsulfanyl)-pyridin-3-yl]-pyrimidin-2-ylamino}-4-methyl-phenyl)-3-trifluoromethyl-benzamide;
N-(4-Methyl-3-{4-[6-(pyridin-3-yloxy)-pyridin-3-yl]-pyrimidin-2-ylamino}-phenyl)-3-trifluoromethyl-benzamide;
N-(4-Methyl-3-{4-[6-(1-methyl-piperidin-4-yloxy)-pyridin-3-yl]-pyrimidin-2-ylamino}-phenyl)-3-trifluoromethyl-benzamide;
N-[3-([4,5']Bipyrimidinyl-2-ylamino)-4-methyl-phenyl]-3-trifluoromethyl-benzamide; and
3-Fluoro-N-[4-methyl-3-(4-pyrazin-2-yl-pyrimidin-2-ylamino)-phenyl]-5-trifluoromethyl-benzamide;
or a pharmaceutically acceptable salt thereof.

4. 3-Dimethylamino-N-[4-methyl-3-(4-pyrazin-2-yl-pyrimidin-2-ylamino)-phenyl]-benzamide, or a pharmaceutically acceptable salt thereof.

5. 3-Diethylamino-N-[4-methyl-3-(4-pyrazin-2-yl-pyrimidin-2-ylamino)-phenyl]-benzamide, or a pharmaceutically acceptable salt thereof.

6. A pharmaceutical composition which is suitable for administration to a warm-blooded animal which is suffering from a tumor disease **characterized by** an aberrant MAP kinase signaling pathway, comprising a compound according to any one of claims 1 to 6, or a pharmaceutically acceptable salt thereof, in an amount that is effective in inhibiting RAF kinase, together with at least one pharmaceutically acceptable carrier.

7. A compound according to any one of claims 1 to 6, or a pharmaceutically acceptable salt thereof, for use in the treatment of a patient having a disease **characterized by** excessive signaling through the MAP kinase pathway.

## Patentansprüche

1. Verbindung mit der Formel (I) 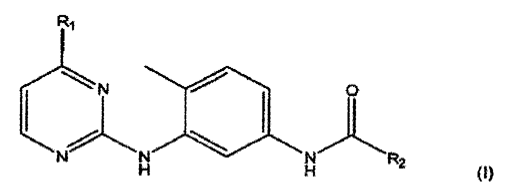 wobei
R₁ ein Phenylradikal, das unsubstituiert oder durch einen Substituenten substituiert oder ein Heteroarytradikal ist, das aus einem Thiazolytradikal, einem Pyrazinylradikal und einem Pyrimidinylradikal ausgewählt ist, wobei jedes hiervon unsubstituiert oder durch einen Substituenten substituiert ist, oder ein 6-substituiertes 3-Pyridylradikal ist; und
R₂ Phenyl ist, das in der 3-position durch Fluor, Halo-Niedrigalkyl, Halo-Niedrigalkoxy oder Hato-Niedrigalkylthio substituiert ist, wobei sich die Bezeichnung "niedrig" auf ein Radikal mit bis zu und einschließlich einem Maximum von 7 Kohlenstoffatomen bezieht,
oder ein N-Oxid oder ein pharmazeutisch verträgliches Salz davon ist.

2. verbindung nach Anspruch 1, wobei R₁ ein Phenyl-, 2-Thiazolyl-, 2-Pyrazinyl-, 5-Pyrimidinyl- oder ein 3-Pyridylradikal ist.

3. Verbindung der Formel 1 nach Anspruch 1, ausgewählt aus der Gruppe, die aus
N-[4-Methyl-3-(4-thiazol-2-yl-pyrimidin-2-ylamino)-phenyl]-3-trifluormethylbenzamid,
N-[4-Methyl-3-(4-pyrazin-2-yl-pyrimidin-2-ylamino)-phenyl]-3-trifluormethylbenzamid;
N-[4-Methyl-3-(4-pyrazin-2-yl-pyrimidin-2-ylamino)-phenyl]-3-(1,1,2,2-tetrafluorethoxy)-benzamid_{;}
3-Difluormethoxy-N-[4-methyl-3-(4-pyrazin-2-yl-pyrimidin-2-ylamino)-phenyl]-benzamid;
N-[4-Methyl-3-(4-pyrazin-2-yl-pyrimidin-2-ylamino)-phenyl]-3-(2,2,2-trifluorethoxy)-benzamid;
N-[4-Methyl-3-(4-pyrazin-2-yl-pyrimidin-2-ylamino)-phenyl]-3-trifluormethylsulfanylbenzamid;
N-{3-[4-(4-Methoxy-phenyl)-pyrimidin-2-ylamino]-4methyl-phenyl}-3-trifluormethylbenzamid;
N-[4-Methyl-3-(4-phenyl-pyrimidin-2-ylamino)-phenyl]-3-trifluormrthyl-benzamid,
N-{3-[4-(3-Methoxy-phenyl)-pyrimidin-2-ylamino]-4-methyl-phenyl}-3-trifluormethylbenzamid;
N-[4-Methyl-3-(4-pyrazin-2-yl-pyrimidin-2-ylamino)-phenyl]-3-trifluormethoxy-benzamid;
N-{3-[4-(3-Ethyl-pyrazin-2-yl)-pyrimidin-2-ylamino]-4-methyl-phenyl}-3-tritluormethylbenzamid;
N-(4-Methyl-3-{4-[6-(3-morpholin-4-yl-proylamino)-pyridin-3-yl]-pyrimidin-2-ylamino}-pheny)-3-trifluormethyl-benzamid;
N-(4-Methyl-3-{4-[6-(pyridin-4-ylamino)-pyridin-3-yl]-pyrimidin-2-ylamino}-phenyl)-3-trifluormethyl-benzamid,
3-Difluormethoxy-N-(3-{4-[6-(-hydroxy-ethylamino)-pyridin-3-yl]-pyrimidin-2-ylamino}-4-methyl-phenyl)-benzamid;
N-{3-[4-(6-Chlorpyridin-3-yl)-pyrimidin-2-ylamino]-4-methyl-phenyl}-3-trifluormethyl-benzamid;
N-{3-[4-(6-Dimethylamino-pyridin-3-yl)-pyrimidin-2-ylamino]-4-methylphenyl}-3-trifluormethyl-benzamid;
N-{3-{4-[6-(2-Methoxy-ethoxy)-pyridin-3-yl]-pyrimidin-2-ylamino}-4-methyl-phenyl)-3-trifluomethyl-benzamid;
N-(4-Methyl-3-{4-[6-(4-methyl-piperazin-1-yl)-pyridin-3-yl]-pyrimidin-2-ylamin}-phenyl)-3-trifluomethyl-benzamid;
N-{4-Methyl-3-[4-(6-methylamino-pyridin-3-yl)-pyrimidin-2-ylamino]-phenyl}-3-trifluormethyl-benzamid;
N-{3-[4-(6-Cyclopropylamino-pyridin-3-yl)-pyrmidin-2-ylamino]-4-methylphenyl}-3-trifluomethyl-benzamid;
N-{3-[4-(6-Cyclopentylamino-pyridin-3-yl)-pyrimidin-2-ylamino]-4-methylphenyl}-3-trifluormethyl-benzamid;
N-{4-Methyl-3-[4-(6-thiomorpholin-4-yl-pyridin-3-yl)-pyrimidin-2-ylamino]-phenyl}-3-trifluormethyl-benzamid;
N-{4-Methyl-3-[4-(6-morpholin-4-yl-pyridin-3-yl)-pyrimidin-2-ylamino]-phenyl}-3-trifluormethyl-benzamid;
N-{3-[4-(4-Hydroxy-3,4,5,6-tetrahydro-2H-[1,2']bipyridinyl-5'-yl)-pyrimidin-2-ylamino]-4-methyl-phenyl}-3-trifluormethyl-benzamid;
N-(3-{4-[6-(3-Diethylamino-propylamino)-pyridin-3-yl]-pyrimidin-2-ylamino}-4-methyl-phenyl)-3-trifluormethyl-benzamid,
N-[4-Methyl-3-(4-{6-[(pyridin-4-ylmethyl)-amino]-pyridin-3-yl]-pyrimidin-2-ylamino)-phenyl]-3-trifluormethyl-benzamid;
N-(3-{4-[6-(2-Methoxy-ethytamino)-pyridin-3-yl]-pyrimidin-2-ylamino}-4-methyl-phenyl)-3-trifluomethyl-benzamid;
N-(3-{4-[6-(2-Hydroxy-propylsulfanyl)-pyridin-3yl]-pyrimidin-2ylamino}-4-methyl-phenyl)-3-trifluormethyl-benzamid;
N-(4-Methyl-3-{4-[6-(pyridin-3-yloxy)-pyridin-3-yl]-pyrimidin-2-ylamino}-phenyl)-3-trifluormethyl-benzamid;
N-(4-Methyl-3-{4-[6-(1-methyl-piperidin-4-yloxy)-pyridin-3-yl]-pyrimidin-2-ylamino}-phenyl)-3-trifluormethyl-benzamid;
N-[3-([4,5']Bipyrimidiyl-2-ylamino)-4-methyl-phenyl]-3-trifluormethyl-benzamid; und
3-Fluor-N-[4-methyl-3-(4-pyrazin-2-yl-pyrimidin-2-ylamino)-phenyl]-5-trifluormethyl-benzamid;
oder einem pharmazeutisch zulässiges Salz davon besteht.

4. 3-Dimethylamino-N-[4-methyl-3-(4-pyrazin-2-yl-pyrimidin-2-ylamino)-pheny]-benzamid, oder ein pharmazeutisch zulässiges Satz davon.

5. 3-Diethylamino-N-[4-methyl-3-(4-pyrazin-2-yl-pyrimidin-2-ylamino)-phenyl]-benzamid, oder ein pharmazeutisch zulässiges Salz davon.

6. Pharmazeutische Zusammensetzung, die zur Verabreichung an ein warmblütiges Lebewesen geeignet ist, das an einer Tumorerkrankung leidet, die durch einen aberrierenden MAP-Kinase-Signatübertragungsweg **gekennzeichnet** ist, wobei die pharmazeutische Zusammensetzung eine Verbindung nach einem der Anspruche 1 bis 5 oder ein pharmazeutisch zulässiges Salz davon in einer Menge, die zur RAF Kinasehemmung wirksam ist, zusammen mit zumindest einem pharmazeutisch zulässigen Träger aufweist.

7. Verbindung nach einem der Ansprüche 1 bis 5 oder ein pharmazeutisch verträgliches Salz davon, zur Verwendung bei der Behandlung eines Patienten mit einer Erkrankung, die durch eine exzessive Signalübertragung durch den MAP-Kinaseweg **gekennzeichnet** ist.

## Revendications

1. Composé de la formule (1) 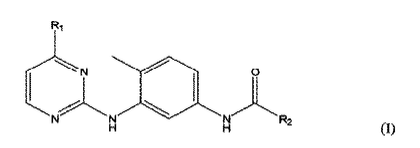
**caractérisé en ce que** R₁ est un radical phényl qui est non substitué ou substitué par un substituant ou un radical hétéroaryl choisi parmi un radical thiazolyl, pyrazenyl, et un radical pyrimidinyl, chacun d'eux étant non substitués ou substitués par un substituant, ou est un 6-substitué 3-pyridyl et R₂ est un phényl qui est substitué dans la région par fluor, alkyl, halo-inférieur, alkoxy halo-inférieur ou thioalkyl halo-inférieur,
Et où le terme « intérieur » se réfère un radical ayant jusqu'à et incluant un maximum de sept atomes de carbone ;
Ou un N-oxyde ou un sel acceptable sur le plan pharmaceutique de celui-ci.

2. Composé selon la revendication 1, **caractérisé en ce que** R₁ est un phényl, 2-thiazolyl, 2-pyrazinyl, 5-pyrimidinyl ou 3- pyridyl radical,

3. Composé selon la formulé 1 de la revendication 1 choisi parmi le groupe consistant en N-[4-Methyl-3-(4-thiazol-2-yl-pyrimidin-2-ylamino)-phenyl]-3-trifluoromethyl-benzamide;
N-[4-Methyl-3-(4-pyrazin-2-yl-pyrimidin-2-ylamino)-phenyl]-3-trifluotomethyl-benzamide;
N-[4-Methyl-3-(4-pyrazin-2-yl-pyrimidin-2-ylamino)-phenyl]-3-(1,1,2,2-tetrafluoroethoxy)-benzamide;
3-Diftuoromethoxy-N-[4-methyt-3-(4-pyrazin-2-yl-pyrimidin-2-ylamino)-phenyl]-benzamide;
N-[4-Methyl-3-(4-pyrazin-2-yl-pyrimidin-2-ylamino)-phenyl]-3-trifluoromethylsulfanyl-benzamide;
N-{3-[4-(4-Methoxy-phenyl)-pyrimidin-2-ylamino]-4-methyl-phenyl}-3-trifluoromethyl-benzamide;
N-[4-Methyl-3-(4-phenyl-pyrimidin-2-ylamino)-phenyl]-3-trifluoromethyl-benzamide;
N-{3-[4-(3-Methoxy-phenyl)-pyrimidin-2-ylamino]-4-methyl-phenyl}-3-trifluoromethyl-benzamide;
N-[4-Methyl-3-(4-pyrazin-2-yl-pyrimidin-2-ylamino)-phenyl]-3-trifluoromethoxy-benzamide
N-{3-[4-(3-Ethyl-pyrazin-2-yl)-pyrimidin-2-ylamino]-4-methyl-phenyl}-3-trifluoromethyl-benzamide;
N-(4-Methyl-3-{4-[6-(3-morpholin)-4-yl-propylamino)-pyrindin-3-yl]-pyrimidin-2ylamino}-phenyl)-3-trifluoromethyl-benzamide;
N-(4-Methyl-3-{4-[6-(pyridin-4-ylamino)-pyridin-3-yl]-pyrimidin-2-ylamino}-phenyl)-3-trifluoromethyl-benzamide;
3-Difluoromethoxy-N-(3-{4-[6-(2-hydroxy-ethylamino)-pyridin-3-yl]-pyrimidin-2-ylamino}-4-methyl-phenyl)-benzamide;
N-{3-[4-(6-Chloro-pyridin-3-yl)-pyrimidin-2-ylamino]-4-methyl-phenyl}-3-trifluoromethyl-benzamide;
N-{3-[4-(6-Dimethylamino-pyridin-3-yl)-pyrimidin-2-ylamino]-4-methyl-phenyl}-3-trifluoromethyl-benzamide;
N-(3-{4-[6-(2-Methoxy-ethoxy)-pyridin-3-yl]-pyrimidin-2-ylamino}-4-methyl-phenyl)-3-trifluoromethyl-benzamide;
N-(4-Methyl-3-{4-[6-(4-methyl-piperazin-1-yl)-pyridin-3-yl]-pyrimidin-2-ylamino}-phenyl)-3-trifluoromethyl-benzamide;
N-{4-Methyl-3-[4-(6-methylamino-pyridin-3-yl)-pyrimidin-2-ylamino]-phenyl}-3-trifluoromethyl-benzamide;
N-{3-[4-(6-Cyclopropylamino-pyridin-3-yl)-pyrimidin-2-ylamino]-4-methyl-phenyl}-3-trifluoromethyl-benzamide;
N-{3-[4-(6-Cyclopentylamino-pyridin-3-yl)-pyrimidin-2-ylamino]-4-methyl-phenyl}-3-trifluoromethyl-benzamide;
N-{4-Methyl-3-[4-(6-thiomorpholin-4yl-pyridin-3-yl)-pyrimidin-2-ylamino]-phenyl}-3-trifluoromethyl-benzamide;
N-(4-Methyl-3-[4-(6-morpholin-4-yl-pyridin-3-yl)-pyrimidin-2-ylamino]-phenyl}-3-trifluoromethyl-benzamide;
N-{3-[4-(4-Hydroxy-3,4,5,6-tetrahydro-2H-[1,2']bipyridinyl-5'-yl)-pyrimidin-2-ylamino]-4-methyl-phenyl}-3-trifluoromethyl-benzamide;
N-(3-{4-[6-(3-Diethyamino-propylamino)-pyridin-3-yl]-pyrimidin-2-ylamino}-4-methyl-phenyl)-3-trifluoromethyl-benzamide;
N-[4-Methyl-3-(4-{6-[(pyridin-4-ylmethyl)-amino]-pyridin-3-yl}pyrimidin-2-ylamino)-phenyl]-3-trifluoromethyl-benzamide;
N-(3-{4-[6-(2-Methoxy-ethylamino)-pyridin-3-yl]-pyrimidin-2-ylamino}-4-methyl-phenyl)-3-trifluoromethyl-benzamide;
N-(3-{4-[6-(2-Hydroxy-propylsulfanyl)-pyridin-3-yl]-pyrimidin-2-ylamino}-4-methyl-phenyl)-3-trifluoromethyl-benzamide;
N-(4-Methyl-3-{4-[6-(pyridin-3-yloxy)-pyridin-3-yl]-pyrimidin-2-ylamino}-phenyl)-3-trifluoromethyl-benzamide;
N-(4-Methyl-3-{4-[6-(1-methyl-piperidin-4-yloxy)-pyridin-3-yl]-pyrimidin-2-ylamino}-phenyl)-3-trifluoromethyl-benzamide;
N-[3-([4,5']Bipyrimidinyl-2-ylamino)-4-methyl-phenyl]-3trifluoromethyl-benzamide; and 3-Fluoro-N-[4-methyl-3-(4-pyrazin-2-yl-pyrimidin-2-ylamino)-phonyl]-5-trifluoromethyl-benzamide;
Ou un sel de celui d, acceptable sur le plan pharmaceutique.

4. Diméthylamino-N-[4-Méthyl-3(4-pyrazin-2yl-pyrimidin-2-ylamino)-phenyl]-benzamide ou un sel de celui ci, acceptable sur le plan pharmaceutique.

5. Diméthylamino-N-[4-Methyl-3-(4-pyrazin-2-yl-pyrimidin-2-ylamino)-phényl]-benzamine, ou un sel de celui ci, acceptable sur le plan pharmaceutique.

6. Composition pharmaceutique qui est adaptée pour être administrée un animal à sang chaud, souffrant d'une tumeur, **caractérisée par** un cheminement signalant kinase aberrant, comprenant un composé selon l'une des revendications 1 à, ou un sel de celui-ci acceptable sur le plan pharmaceutique, dans une quotité qui est effective pour inhiber kinase RAF, ensemble avec au moins un porteur acceptable sur le plan pharmaceutique.

7. Composé selon l'une des revendications 1 à 6, ou un sel de celui acceptable sur le plan pharmaceutique, pour utilisation dans le traitement d'un patient ayant une maladie **caractérisée par** des symptômes excessifs par le cheminement kinase MAP,
